# EUROPEAN PATENT APPLICATION

(11) **EP 2 532 362 A1**
(43) Date of publication of application: **12.12.2012**
(21) Application number: 12180148.4
(22) Date of filing: 05.12.2007
(51) Int. Cl.: A61K 39/145, A61P 31/16, C12N 7/00

(54) **Vaccines including antigen from four strains of influenza virus**

(30) Priority: 06.12.2006 US 873815 P
(62) Divisional of application: 07866632.8
(71) Applicant: Novartis AG, 4056 Basel (CH)
(72) Inventor: Tsai, Theodore F., Boston, MA Massachusetts 02139 (US)
(74) Representative: Carpmaels & Ransford

(57) **Abstract**

Vaccines of the invention include at least four influenza virus strains. In some embodiments, the vaccines are produced in cell culture rather than in eggs. In some embodiments, the vaccines include an adjuvant. In some embodiments, the vaccines are not split or whole virion vaccines, but are live or purified glycoprotein vaccines. In some embodiments, the vaccines contain substantially the same mass of hemagglutinin (HA) for each of the influenza virus strains. In some embodiments, the four strains will include two influenza A virus strains and two influenza B virus strains ('A-A-B-B'). In other embodiments, the four strains will include three influenza A virus strains and one influenza B virus strain (`A-A-A-B').

## Description

### TECHNICAL FIELD

This invention is in the field of vaccines for protecting against influenza virus infection, and in particular vaccines that include antigens derived from more than three viral strains.

### DISCLOSURE OF THE INVENTION

The applicant has realized that many deficiencies in current trivalent vaccines are caused by the use of eggs during their manufacture. Egg-based manufacturing is not well-suited for increasing the amount of antigen required in a particular season, in terms of both lead time and capacity. Current egg-based manufacturing requires viruses to be adapted to growth in eggs, which both adds a time-consuming phase at the beginning of manufacture and introduces a selection pressure which reduces the match between vaccine strains and circulating strains. Moreover, influenza B virus strains do not grow well in eggs, and high-growth reassortants are not available. The absolute requirement for egg growth in current procedures is well demonstrated by the H3N2 mismatch in the 2003/04 season, which mainly arose because the relevant Fuji-like strains had originally not been isolated using eggs. To overcome these deficiencies, cell culture can be used for viral growth instead of eggs. In some embodiments, therefore, vaccines of the invention are produced from viruses grown in cell culture rather than in eggs.

The inability of egg-based technology to increase the amount of antigen produced in a particular season could also be addressed by reducing the amount of antigen in a vaccine. One way of reducing the amount is to use an adjuvant. In some embodiments, therefore, vaccines of the invention include an adjuvant.

In some embodiments, the vaccines contain substantially the same mass of hemagglutinin (HA) for each of the influenza virus strains (*e.g.* about 1:1:1:1). The mass of HA per strain may about 15µg per dose, or in some embodiments may be less than 15µg per dose (*e.g.* less than 10µg per dose) or more than 15µg per dose (*e.g.* >20µg/dose, >25µg/dose, *etc.,* such as about 30µg/dose). In other embodiments the vaccines contain different masses of HA for different strains.

In some embodiments, the vaccines are not split or whole virion inactivated vaccines, but are live or purified glycoprotein vaccines.

Thus the invention provides a vaccine comprising antigen from at least four strains of influenza virus, wherein at least one of the strains was grown in cell culture. Preferably all strains were grown in cell culture.

The invention also provides a vaccine comprising antigen from at least four strains of influenza virus, wherein the vaccine is does not contain ovalbumin. The composition can also be free from other egg proteins (*e.g.* ovomucoid) and from chicken DNA.

The invention also provides a method for preparing a vaccine comprising the steps of: (i) growing four different strains of influenza virus in cell culture; (ii) preparing an antigen composition from each of the viruses grown in step (i); and (iii) combining the antigen compositions (*e.g.* with a pharmaceutical carrier), to give the vaccine.

The invention also provides a vaccine comprising antigen from at least four strains of influenza virus, and including an adjuvant.

The invention also provides a method for preparing a vaccine comprising the steps of: (i) growing four different strains of influenza virus; (ii) preparing an antigen composition from each of the viruses grown in step (i); and (iii) combining the antigen compositions with an adjuvant and a pharmaceutical carrier, to give the vaccine.

The invention also provides a vaccine comprising antigen from at least four strains of influenza virus, wherein the antigens are neither split virions nor whole virions. The antigens may be live influenza virus, or may be purified glycoproteins i. e. the vaccine will comprise purified hemagglutinin from at least four strains of influenza virus.

The invention also provides a method for preparing a vaccine comprising the steps of: (i) growing four different strains of influenza virus; (ii) preparing an antigen composition from each of the viruses grown in step (i), which antigen composition is neither a split virion nor a whole virion; and (iii) combining the antigen compositions with a pharmaceutical carrier, to give the vaccine.

The invention also provides a vaccine comprising antigen from at least four strains of influenza virus, wherein the vaccine contains substantially the same mass of hemagglutinin (HA) for each of the influenza virus strains. The mass of HA for each of the strains will be within 10% of the mean mass of HA per strain. The mass is preferably less than 15µg HA per strain.

The invention also provides a vaccine comprising antigen from at least four strains of influenza virus, wherein the vaccine contains *a* µg of hemagglutinin (HA) from a first strain, *b* µg of HA from a second strain, *c* µg of HA from a third strain, and *d* µg of HA from a fourth strain, wherein *a* and *b* are substantially the same and *c* and *d* are substantially the same, but *a* and *c* are substantially different. The values of *a* and *b* will be within 10% of the mean of *a* and *b* ('the *a*/*b* mean'). The values of *c* and *d* will be within 10% of the mean of *c* and *d* ('the *c*/*d* mean'). The *a*/*b* mean and the *c*/*d* mean will differ by at least 25% of the lower of the *a*/*b* mean and the *c*/*d* mean (*e.g.* by at least 33%, by at least 40%, by at least 50%).

The invention also provides a vaccine comprising antigen from at least four strains of influenza virus, wherein the vaccine contains *a* µg of hemagglutinin (HA) from a first strain, *b* µg of HA from a second strain, *c* µg of HA from a third strain, and *d* µg of HA from a fourth strain, wherein *a*, *b* and *c* are substantially the same as each other but are substantially different from *d*. The values of *a*, *b* and *c* will be within 10% of the mean of *a*, *b* and *c* ('the *a*/*b*/*c* mean'). The value of *d* will differ from the *a*/*b*/*c* mean by at least 25% of the *a*/*b*/*c* mean (*e.g.* by at least 33%, by at least 40%, by at least 50%). The invention also provides a vaccine comprising antigen from at least four strains of influenza virus, wherein the vaccine has a mean amount of HA per strain of *x* µg, and wherein either (a) the amount of HA for two of the four strains is at least 10% below x and the amount of HA for the other two strains is at least 10% above *x*, or (b) the amount of HA for three of the four strains is at least 10% below x and the amount of HA for the other strain is at least 10% above *x*, or (c) the amount of HA for three of the four strains is at least 10% above x and the amount of HA for the other strain is at least 10% below *x*. The 'at least 10%' may be at least 25%, at least 33%, at least 40%, at least 50%, *etc.*

The invention also provides a method for preparing a vaccine comprising the steps of: (i) growing four different strains of influenza virus; (ii) preparing an antigen composition from each of the viruses grown in step (i), wherein the antigen composition includes hemagglutinin; and (iii) combining the antigen compositions with a pharmaceutical carrier, to give the vaccine, in proportions that give substantially the same mass of hemagglutinin for each of the influenza virus strains in said vaccine.

In some embodiments, the four strains will include two influenza A virus strains and two influenza B virus strains. In other embodiments, the four strains will include three influenza A virus strains and one influenza B virus strain.

### Strain selection

Influenza virus strains for use in vaccines change from season to season. In the current inter-pandemic period, trivalent vaccines include two influenza A strains (H1N1 and H3N2) and one influenza B strain. Vaccines of the invention include at least four influenza virus strains. The different strains will typically be grown separately and then mixed after the viruses have been harvested and antigens have been prepared. Thus a process of the invention may include the step of mixing antigens from more than one influenza strain.

In some embodiments, the four strains will include two influenza A virus strains and two influenza B virus strains ('A-A-B-B'). In other embodiments, the four strains will include three influenza A virus strains and one influenza B virus strain ('A-A-A-B').

Influenza A virus currently displays sixteen HA subtypes: H1, H2, H3, H4, H5, H6, H7, H8, H9, H10, H11, H12, H13, H14, H15 and H16. The invention may protect against one or more of influenza A virus NA subtypes N1, N2, N3, N4, N5, N6, N7, N8 or N9. In vaccines including only two influenza A virus strains, these will usually be one H1 strain (*e.g.* a H1N1 strain) and one H3 strain (*e.g.* a H3N2 strain). In some embodiments, however, there may be one pandemic strain and one H1 strain, or one pandemic strain and one H3 strain. A 4-valent influenza vaccine may include a H1N1 strain, a H3N2 strain, a H5 strain (*e.g.* a H5N1 strain) and an influenza B strain.

In vaccines including three influenza A virus strains, these will usually be one H1 strain (*e.g.* a H1N1 strain) and two H3 strains (*e.g.* two H3N2 strains). The two H3 strains will have antigenically distinct HA proteins *e.g.* one H3N2 strain that cross-reacts with A/Moscow/10/99 and one H3N2 strain that cross-reacts with A/Fujian/411/2002. The two H3 strains may be from different clades (clades A, B and C of H3N2 strains are disclosed in reference 1). In some embodiments, however, one of these strains (*i.e.* H1, or one of the two H3 strains) may be replaced by a pandemic strain.

Characteristics of a pandemic influenza strain are: (a) it contains a new hemagglutinin compared to the hemagglutinins in currently-circulating human strains, *i.e.* one that has not been evident in the human population for over a decade (*e.g.* H2), or has not previously been seen at all in the human population (*e.g.* H5, H6 or H9, that have generally been found only in bird populations), such that the vaccine recipient and the general human population are immunologically naïve to the strain's hemagglutinin; (b) it is capable of being transmitted horizontally in the human population; and (c) it is pathogenic to humans. Pandemic strains H2, H5, H7 or H9 subtype strains *e.g.* H5N1, H5N3, H9N2, H2N2, H7N1 and H7N7 strains. Within the H5 subtype, a virus may fall into HA clade 1, HA clade 1', HA clade 2 or HA clade 3 [2], with clades 1 and 3 being particularly relevant.

Influenza B virus currently does not display different HA subtypes, but influenza B virus strains do fall into two distinct lineages. These lineages emerged in the late 1980s and have HAs which can be antigenically and/or genetically distinguished from each other [3]. Current influenza B virus strains are either B/Victoria/2/87-like or B/Yamagata/16/88-like. Where a vaccine of the invention includes two influenza B strains, one B/Victoria/2/87-like strain and one B/Yamagata/16/88-like strain will be included. These strains are usually distinguished antigenically, but differences in amino acid sequences have also been described for distinguishing the two lineages *e.g.* B/Yamagata/16/88-like strains often (but not always) have HA proteins with deletions at amino acid residue 164, numbered relative to the 'Lee40' HA sequence [4].

Thus preferred A-A-B-B vaccines of the invention will include antigens (preferably hemagglutinins) from: (i) a H1N1 strain; (ii)a H3N2 strain; (iii) a B/Victoria/2/87-like strain; and (iv) B/Yamagata/16/88-like strain.

In some embodiments of the invention where antigens are present from two or more influenza B virus strains, at least two of the influenza B virus strains may have distinct hemagglutinins but related neuraminidases. For instance, they may both have a B/Victoria/2/87-like neuraminidase [5] or may both have a B/Yamagata/16/88-like neuraminidase. For instance, two B/Victoria/2/87-like neuraminidases may both have one or more of the following sequence characteristics: (1) not a serine at residue 27, but preferably a leucine; (2) not a glutamate at residue 44, but preferably a lysine; (3) not a threonine at residue 46, but preferably an isoleucine; (4) not a proline at residue 51, but preferably a serine; (5) not an arginine at residue 65, but preferably a histidine; (6) not a glycine at residue 70, but preferably a glutamate; (7) not a leucine at residue 73, but preferably a phenylalanine; and/or (8) not a proline at residue 88, but preferably a glutamine. Similarly, in some embodiments the neuraminidase may have a deletion at residue 43, or it may have a threonine; a deletion at residue 43, arising from a trinucleotide deletion in the NA gene, has been reported as a characteristic of B/Victoria/2/87-like strains, although recent strains have regained Thr-43 [5]. Conversely, of course, the opposite characteristics may be shared by two B/Yamagata/16/88-like neuraminidases *e.g.* S27, E44, T46, P51, R65, G70, L73, and/or P88. These amino acids are numbered relative to the 'Lee40' neuraminidase sequence [6]. Thus a A-A-B-B vaccine of the invention may use two B strains that are antigenically distinct for HA (one B/Yamagata/16/88-like, one B/Victoria/2/87-like), but are related for NA (both B/Yamagata/16/88-like, or both B/Victoria/2/87-like).

Preferred A-A-A-B vaccines of the invention will include antigens (preferably hemagglutinins) from: (i) a H1N1 strain; (ii)a A/Moscow/10/99-like H3N2 strain; (iii) a A/Fujian/411/2002-like H3N2 strain; and (iv) an influenza B virus strain, which may be B/Victoria/2/87-like or B/Yamagata/16/88-like.

The invention is not restricted to 4-valent vaccines, and encompasses 5-valent, 6-valent, 7-valent, *etc.* vaccines. An example 5-valent vaccine may include three influenza A strains (*e.g.* one H1 strain and two H3 strains, as discussed above) plus two influenza B strains. For example, an A-A-A-B-B vaccine may include antigens (preferably hemagglutinins) from: (i) a H1N1 strain; (ii) a A/Moscow/10/99-like H3N2 strain; (iii) a A/Fujian/411/2002-like H3N2 strain; (iv) a B/Victoria/2/87-like strain; and (v) a B/Yamagata/16/88-like strain. Another A-A-A-B-B vaccine may include antigens (preferably hemagglutinins) from: (i) a H1N1 strain; (ii) a H3N2 strain; (iii) a H5 influenza A virus strain, such as a H5N1 strain; (iv) a B/Victoria/2/87-like strain; and (v) a B/Yamagata/16/88-like strain. An A-A-A-A-B vaccine may include antigens (preferably hemagglutinins) from: (i) a H1N1 strain; (ii) a A/Moscow/10/99-like H3N2 strain; (iii) a A/Fujian/411/2002-like H3N2 strain; (iv) a H5 influenza A virus strain, such as a H5N1 strain; and (v) an influenza B virus strain. An A-A-A-A-B-B vaccine may include antigens (preferably hemagglutinins) from: (i) a H1N1 strain; (ii) a A/Moscow/10/99-like H3N2 strain; (iii) a A/Fujian/411/2002-like H3N2 strain; (iv) a H5 influenza A virus strain, such as a H5N1 strain; (v) a B/Victoria/2/87-like strain; and (vi) a B/Yamagata/16/88-like strain.

An influenza virus used with the invention may be a reassortant strain, and may have been obtained by reverse genetics techniques. Reverse genetics techniques [*e.g.* 7-11] allow influenza viruses with desired genome segments to be prepared *in vitro* using plasmids. Typically, it involves expressing (a) DNA molecules that encode desired viral RNA molecules *e.g.* from polI promoters or bacteriophage RNA polymerase promoters, and (b) DNA molecules that encode viral proteins *e.g.* from polII promoters, such that expression of both types of DNA in a cell leads to assembly of a complete intact infectious virion. The DNA preferably provides all of the viral RNA and proteins, but it is also possible to use a helper virus to provide some of the RNA and proteins. Plasmid-based methods using separate plasmids for producing each viral RNA can be used [12-14], and these methods will also involve the use of plasmids to express all or some (*e.g.* just the PB1, PB2, PA and NP proteins) of the viral proteins, with up to 12 plasmids being used in some methods. To reduce the number of plasmids needed, a recent approach [15] combines a plurality of RNA polymerase I transcription cassettes (for viral RNA synthesis) on the same plasmid (*e.g.* sequences encoding 1, 2, 3, 4, 5, 6, 7 or all 8 influenza A vRNA segments), and a plurality of protein-coding regions with RNA polymerase II promoters on another plasmid (*e.g.* sequences encoding 1, 2, 3, 4, 5, 6, 7 or all 8 influenza A mRNA transcripts). Preferred aspects of the reference 15 method involve: (a) PB1, PB2 and PA mRNA-encoding regions on a single plasmid; and (b) all 8 vRNA-encoding segments on a single plasmid. Including the NA and HA segments on one plasmid and the six other segments on another plasmid can also facilitate matters.

As an alternative to using polI promoters to encode the viral RNA segments, it is possible to use bacteriophage polymerase promoters [16]. For instance, promoters for the SP6, T3 or T7 polymerases can conveniently be used. Because of the species-specificity of polI promoters, bacteriophage polymerase promoters can be more convenient for many cell types (*e.g.* MDCK), although a cell must also be transfected with a plasmid encoding the exogenous polymerase enzyme.

In other techniques it is possible to use dual polI and polII promoters to simultaneously code for the viral RNAs and for expressible mRNAs from a single template [17,18].

Thus an influenza A virus may include one or more RNA segments from a A/PR/8/34 virus (typically 6 segments from A/PR/8/34, with the HA and N segments being from a vaccine strain, i.e. a 6:2 reassortant). It may also include one or more RNA segments from a A/WSN/33 virus, or from any other virus strain useful for generating reassortant viruses for vaccine preparation. An influenza A virus may include fewer than 6 (*i.e.* 0, 1, 2, 3, 4 or 5) viral segments from an AA/6/60 influenza virus (A/Ann Arbor/6/60). An influenza B virus may include fewer than 6 (*i.e.* 0, 1, 2, 3, 4 or 5) viral segments from an AA/1/66 influenza virus (B/Ann Arbor/1/66). Typically, the invention protects against a strain that is capable of human-to-human transmission, and so the strain's genome will usually include at least one RNA segment that originated in a mammalian (*e.g.* in a human) influenza virus. It may include NS segment that originated in an avian influenza virus.

Strains whose antigens can be included in the compositions may be resistant to antiviral therapy (*e.g.* resistant to oseltamivir [19] and/or zanamivir), including resistant pandemic strains [20].

Particularly useful strains are those that have not been passaged through eggs at any stage between isolation from a patient and replication in a cell culture system, inclusive. The use exclusively of MDCK cells for all steps from isolation to virus replication is one preferred embodiment of the invention.

In some embodiments, strains used with the invention will thus have hemagglutinin with a binding preference for oligosaccharides with a Sia(α2,6)Gal terminal disaccharide compared to oligosaccharides with a Sia(α2,3)Gal terminal disaccharide. Human influenza viruses bind to receptor oligosaccharides having a Sia(α2,6)Gal terminal disaccharide (sialic acid linked α-2,6 to galactose), but eggs and Vero cells have receptor oligosaccharides with a Sia(α2,3)Gal terminal disaccharide. Growth of human influenza viruses in cells such as MDCK provides selection pressure on hemagglutinin to maintain the native Sia(α2,6)Gal binding, unlike egg passaging.

To determine if a virus has a binding preference for oligosaccharides with a Sia(α2,6)Gal terminal disaccharide compared to oligosaccharides with a Sia(α2,3)Gal terminal disaccharide, various assays can be used. For instance, reference 21 describes a solid-phase enzyme-linked assay for influenza virus receptor-binding activity which gives sensitive and quantitative measurements of affinity constants. Reference 22 used a solid-phase assay in which binding of viruses to two different sialylglycoproteins was assessed (ovomucoid, with Sia(α2,3)Gal determinants; and pig α₂-macroglobulin, which Sia(α2,6)Gal determinants), and also describes an assay in which the binding of virus was assessed against two receptor analogs: free sialic acid (Neu5Ac) and 3'-sialyllactose (Neu5Acα2-3Ga1β1-4Glc). Reference 23 reports an assay using a glycan array which was able to clearly differentiate receptor preferences for α2,3 or α2,6 linkages. Reference 24 reports an assay based on agglutination of human erythrocytes enzymatically modified to contain either Sia(α2,6)Gal or Sia(α2,3)Gal. Depending on the type of assay, it may be performed directly with the virus itself, or can be performed indirectly with hemagglutinin purified from the virus.

In some embodiments influenza strains used with the invention have glycoproteins (including hemagglutinin) with a different glycosylation pattern from egg-derived viruses. Thus the glycoproteins will include glycoforms that are not seen in chicken eggs.

In some embodiments, the invention does not use a combination of A/Singapore/6/86(H1N1), A/Beijing/353/89(H3N2), B/Beijing/1/87, and B/Panama/45/90 strains (and in particular not a combination of split antigens from these four strains, and/or not with 15µg HA per strain). In other embodiments, the invention does not use a combination of A/Taiwan/1/86(H1N1), A/Guizhou/54/89(H3N2), B/Beijing/1/87 and B/Yamagata/16/88 strains (and in particular not a combination of whole virions from these four strains).

### Vaccine preparation

Various forms of influenza virus vaccine are currently available, and vaccines are generally based either on live virus or on inactivated virus. Inactivated vaccines may be based on whole virions, 'split' virions, or on purified surface antigens. Influenza antigens can also be presented in the form of virosomes. The invention can used with any of these types of vaccine.

Existing live vaccines include MedImmune's FLUMIST™ product (trivalent live virus). Vaccine is prepared by a process that comprises growing the virus on a suitable substrate and then purifying virions from virion-containing fluids. For example, the fluids may be clarified by centrifugation, and stabilized with buffer (*e.g.* containing sucrose, potassium phosphate, and monosodium glutamate).

Where an inactivated virus is used, the vaccine may comprise whole virion, split virion, or purified surface antigens (including hemagglutinin and, usually, also including neuraminidase). Chemical means for inactivating a virus include treatment with an effective amount of one or more of the following agents: detergents, formaldehyde, β-propiolactone, methylene blue, psoralen, carboxyfullerene (C60), binary ethylamine, acetyl ethyleneimine, or combinations thereof. Non-chemical methods of viral inactivation are known in the art, such as for example UV light or gamma irradiation.

Virions can be harvested from virus-containing fluids by various methods. For example, a purification process may involve zonal centrifugation using a linear sucrose gradient solution that includes detergent to disrupt the virions. Antigens may then be purified, after optional dilution, by diafiltration.

Split virions are obtained by treating purified virions with detergents (*e.g.* ethyl ether, polysorbate 80, deoxycholate, tri-*N*-butyl phosphate, Triton X-100, Triton N101, cetyltrimethylammonium bromide, Tergitol NP9, *etc.*) to produce subvirion preparations, including the 'Tween-ether' splitting process. Methods of splitting influenza viruses are well known in the art *e.g.* see refs. 25-30, *etc.* Splitting of the virus is typically carried out by disrupting or fragmenting whole virus, whether infectious or non-infectious with a disrupting concentration of a splitting agent. The disruption results in a full or partial solubilisation of the virus proteins, altering the integrity of the virus. Preferred splitting agents are non-ionic and ionic (*e.g.* cationic) surfactants *e.g.* alkylglycosides, alkylthioglycosides, acyl sugars, sulphobetaines, betains, polyoxyethylenealkylethers, N,N-dialkyl-Glucamides, Hecameg, alkylphenoxy-polyethoxyethanols, quaternary ammonium compounds, sarcosyl, CTABs (cetyl trimethyl ammonium bromides), tri-*N*-butyl phosphate, Cetavlon, myristyltrimethylammonium salts, lipofectin, lipofectamine, and DOT-MA, the octyl- or nonylphenoxy polyoxyethanols (*e.g.* the Triton surfactants, such as Triton X-100 or Triton N101), polyoxyethylene sorbitan esters (the Tween surfactants), polyoxyethylene ethers, polyoxyethlene esters, *etc.* One useful splitting procedure uses the consecutive effects of sodium deoxycholate and formaldehyde, and splitting can take place during initial virion purification (e.g. in a sucrose density gradient solution). Thus a splitting process can involve clarification of the virion-containing material (to remove non-virion material), concentration of the harvested virions (*e.g.* using an adsorption method, such as CaHPO₄ adsorption), separation of whole virions from non-virion material, splitting of virions using a splitting agent in a density gradient centrifugation step (*e.g.* using a sucrose gradient that contains a splitting agent such as sodium deoxycholate), and then filtration (*e.g.* ultrafiltration) to remove undesired materials. Split virions can usefully be resuspended in sodium phosphate-buffered isotonic sodium chloride solution. The BEGRIVAC™, FLUARIX™, FLUZONE™ and FLUSHIELD™ products are split vaccines.

Purified surface antigen vaccines comprise the influenza surface antigens haemagglutinin and, typically, also neuraminidase. Processes for preparing these proteins in purified form are well known in the art. The FLUVIRIN™, AGRIPPAL™ and INFLUVAC™ products are subunit vaccines.

Another form of inactivated influenza antigen is the virosome [31] (nucleic acid free viral-like liposomal particles). Virosomes can be prepared by solubilization of influenza virus with a detergent followed by removal of the nucleocapsid and reconstitution of the membrane containing the viral glycoproteins. An alternative method for preparing virosomes involves adding viral membraneglycoproteins to excess amounts of phospholipids, to give liposomes with viral proteins in their membrane. The invention can be used to store bulk virosomes. as in the INFLEXAL V™ and INVAVAC™ products.

The influenza virus may be attenuated. The influenza virus may be temperature-sensitive. The influenza virus may be cold-adapted. These three features are particularly useful when using live virus as an antigen.

HA is the main immunogen in current inactivated influenza vaccines, and vaccine doses are standardised by reference to HA levels, typically measured by SRID. Existing vaccines typically contain about 15µg of HA per strain, although lower doses can be used *e.g.* for children, or in pandemic situations, or when using an adjuvant. Fractional doses such as ½ (*i.e.* 7.5µg HA per strain), ¼ and ⅛ have been used [68,69], as have higher doses (*e.g.* 3x or 9x doses [32,33]). Thus vaccines may include between 0.1 and 150µg of HA per influenza strain, preferably between 0.1 and 50µg *e.g.* 0.1-20µg, 0.1-15µg, 0.1-10µg, 0.1-7.5µg, 0.5-5µg, *etc.* Particular doses include *e.g.* about 45, about 30, about 15, about 10, about 7.5, about 5, about 3.8, about 1.9, about 1.5, *etc.* per strain. It is preferred to use substantially the same mass of HA for each strain included in the vaccine *e.g.* such that the HA mass for each strain is within 10% of the mean HA mass per strain, and preferably within 5% of the mean.

For live vaccines, dosing is measured by median tissue culture infectious dose (TCID₅₀) rather than HA content, and a TCID₅₀ of between 10⁶ and 10⁸ (preferably between 10^{6.5}-10^{7.5}) per strain is typical.

Strains used with the invention may have a natural HA as found in a wild-type virus, or a modified HA. For instance, it is known to modify HA to remove determinants (e.g. hyper-basic regions around the HA1/HA2 cleavage site) that cause a virus to be highly pathogenic in avian species. The HAs of influenza B viruses used with the invention preferably have Asn at amino acid 197, providing a glycosylation site [34].

### Cell lines

Rather than use SPF eggs as the substrate for viral growth, it is preferred to use cell lines that support influenza virus replication. The cell line will typically be of mammalian origin. Suitable mammalian cells of origin include, but are not limited to, hamster, cattle, primate (including humans and monkeys) and dog cells, although the use of primate cells is not preferred. Various cell types may be used, such as kidney cells, fibroblasts, retinal cells, lung cells, *etc.* Examples of suitable hamster cells are the cell lines having the names BHK21 or HKCC. Suitable monkey cells are *e.g.* African green monkey cells, such as kidney cells as in the Vero cell line [35-37]. Suitable dog cells are *e.g.* kidney cells, as in the CLDK and MDCK cell lines.

Thus suitable cell lines include, but are not limited to: MDCK; CHO; CLDK; HKCC; 293T; BHK; Vero; MRC-5; PER.C6 [38]; FRhL2; WI-38; *etc.* Suitable cell lines are widely available *e.g.* from the American Type Cell Culture (ATCC) collection [39], from the Coriell Cell Repositories [40], or from the European Collection of Cell Cultures (ECACC). For example, the ATCC supplies various different Vero cells under catalog numbers CCL-81, CCL-81.2, CRL-1586 and CRL-1587, and it supplies MDCK cells under catalog number CCL-34. PER.C6 is available from the ECACC under deposit number 96022940.

The most preferred cell lines are those with mammalian-type glycosylation. As a less-preferred alternative to mammalian cell lines, virus can be grown on avian cell lines [*e.g.* refs. 41-43], including cell lines derived from ducks (*e.g.* duck retina) or hens. Examples of avian cell lines include avian embryonic stem cells [41,44] and duck retina cells [42]. Suitable avian embryonic stem cells, include the EBx cell line derived from chicken embryonic stem cells, EB45, EB14, and EB14-074 [45]. Chicken embryo fibroblasts (CEF) may also be used. Rather than using avian cells, however, the use of mammalian cells means that vaccines can be free from avian DNA and egg proteins (such as ovalbumin and ovomucoid), thereby reducing allergenicity.

The most preferred cell lines for growing influenza viruses are MDCK cell lines [46-49], derived from Madin Darby canine kidney. The original MDCK cell line is available from the ATCC as CCL-34, but derivatives of this cell line may also be used. For instance, reference 46 discloses a MDCK cell line that was adapted for growth in suspension culture ('MDCK 33016', deposited as DSM ACC 2219). Similarly, reference 50 discloses a MDCK-derived cell line that grows in suspension in serum-free culture ('B-702', deposited as FERM BP-7449). Reference 51 discloses non-tumorigenic MDCK cells, including 'MDCK-S' (ATCC PTA-6500), 'MDCK-SF101' (ATCC PTA-6501), 'MDCK-SF102' (ATCC PTA-6502) and 'MDCK-SF103' (PTA-6503). Reference 52 discloses MDCK cell lines with high susceptibility to infection, including 'MDCK.5F1' cells (ATCC CRL-12042). Any of these MDCK cell lines can be used.

Virus may be grown on cells in adherent culture or in suspension. Microcarrier cultures can also be used. In some embodiments, the cells may thus be adapted for growth in suspension.

Cell lines are preferably grown in serum-free culture media and/or protein free media. A medium is referred to as a serum-free medium in the context of the present invention in which there are no additives from serum of human or animal origin. The cells growing in such cultures naturally contain proteins themselves, but a protein-free medium is understood to mean one in which multiplication of the cells occurs with exclusion of proteins, growth factors, other protein additives and non-serum proteins, but can optionally include proteins such as trypsin or other proteases that may be necessary for viral growth.

Cell lines supporting influenza virus replication are preferably grown below 37°C [53] (*e.g.* 30-36°C, or at about 30°C, 31°C, 32°C, 33°C, 34°C, 35°C, 36°C) during viral replication.

Methods for propagating influenza virus in cultured cells generally includes the steps of inoculating a culture of cells with an inoculum of the strain to be grown, cultivating the infected cells for a desired time period for virus propagation, such as for example as determined by virus titer or antigen expression (*e.g.* between 24 and 168 hours after inoculation) and collecting the propagated virus. The cultured cells are inoculated with a virus (measured by PFU or TCID₅₀) to cell ratio of 1:500 to 1:1, preferably 1:100 to 1:5, more preferably 1:50 to 1:10. The virus is added to a suspension of the cells or is applied to a monolayer of the cells, and the virus is absorbed on the cells for at least 60 minutes but usually less than 300 minutes, preferably between 90 and 240 minutes at 25°C to 40°C, preferably 28°C to 37°C. The infected cell culture (e.g. monolayers) may be removed either by freeze-thawing or by enzymatic action to increase the viral content of the harvested culture supernatants. The harvested fluids are then either inactivated or stored frozen. Cultured cells may be infected at a multiplicity of infection ("m.o.i.") of about 0.0001 to 10, preferably 0.002 to 5, more preferably to 0.001 to 2. Still more preferably, the cells are infected at a m.o.i of about 0.01. Infected cells may be harvested 30 to 60 hours post infection. Preferably, the cells are harvested 34 to 48 hours post infection. Still more preferably, the cells are harvested 38 to 40 hours post infection. Proteases (typically trypsin) are generally added during cell culture to allow viral release, and the proteases can be added at any suitable stage during the culture e.g. before inoculation, at the same time as inoculation, or after inoculation [53].

In preferred embodiments, particularly with MDCK cells, a cell line is not passaged from the master working cell bank beyond 40 population-doubling levels.

The viral inoculum and the viral culture are preferably free from (*i.e.* will have been tested for and given a negative result for contamination by) herpes simplex virus, respiratory syncytial virus, parainfluenza virus 3, SARS coronavirus, adenovirus, rhinovirus, reoviruses, polyomaviruses, birnaviruses, circoviruses, and/or parvoviruses [54]. Absence of herpes simplex viruses is particularly preferred.

### Host cell DNA

Where virus has been grown on a cell line then it is standard practice to minimize the amount of residual cell line DNA in the final vaccine, in order to minimize any oncogenic activity of the DNA.

Thus a vaccine composition prepared according to the invention preferably contains less than 10ng (preferably less than 1ng, and more preferably less than 100pg) of residual host cell DNA per dose, although trace amounts of host cell DNA may be present.

Vaccines containing <10ng (*e.g.* <1ng, <100pg) host cell DNA per 15µg of haemagglutinin are preferred, as are vaccines containing <10ng (*e.g.* <1ng, <100pg) host cell DNA per 0.25ml volume. Vaccines containing <10ng (*e.g.* <1ng, <100pg) host cell DNA per 50µg of haemagglutinin are more preferred, as are vaccines containing <10ng (*e.g.* <1ng, <100pg) host cell DNA per 0.5ml volume.

It is preferred that the average length of any residual host cell DNA is less than 500bp *e.g.* less than 400bp, less than 300bp, less than 200bp, less than 100bp, *etc.*

Contaminating DNA can be removed during vaccine preparation using standard purification procedures *e.g.* chromatography, *etc.* Removal of residual host cell DNA can be enhanced by nuclease treatment *e.g.* by using a DNase. A convenient method for reducing host cell DNA contamination is disclosed in references 55 & 56, involving a two-step treatment, first using a DNase (*e.g.* Benzonase), which may be used during viral growth, and then a cationic detergent (*e.g.* CTAB), which may be used during virion disruption. Removal by β-propiolactone treatment can also be used.

Measurement of residual host cell DNA is now a routine regulatory requirement for biologicals and is within the normal capabilities of the skilled person. The assay used to measure DNA will typically be a validated assay [57,58]. The performance characteristics of a validated assay can be described in mathematical and quantifiable terms, and its possible sources of error will have been identified. The assay will generally have been tested for characteristics such as accuracy, precision, specificity. Once an assay has been calibrated (*e.g.* against known standard quantities of host cell DNA) and tested then quantitative DNA measurements can be routinely performed. Three main techniques for DNA quantification can be used: hybridization methods, such as Southern blots or slot blots [59]; immunoassay methods, such as the Threshold™ System [60]; and quantitative PCR [61]. These methods are all familiar to the skilled person, although the precise characteristics of each method may depend on the host cell in question *e.g.* the choice of probes for hybridization, the choice of primers and/or probes for amplification, *etc.* The Threshold™ system from *Molecular Devices* is a quantitative assay for picogram levels of total DNA, and has been used for monitoring levels of contaminating DNA in biopharmaceuticals [60]. A typical assay involves non-sequence-specific formation of a reaction complex between a biotinylated ssDNA binding protein, a urease-conjugated anti-ssDNA antibody, and DNA. All assay components are included in the complete Total DNA Assay Kit available from the manufacturer. Various commercial manufacturers offer quantitative PCR assays for detecting residual host cell DNA e.g. AppTec™ Laboratory Services, BioReliance™, Althea Technologies, *etc.* A comparison of a chemiluminescent hybridisation assay and the total DNA Threshold™ system for measuring host cell DNA contamination of a human viral vaccine can be found in reference 62.

### Pharmaceutical compositions

Compositions of the invention are pharmaceutically acceptable. They usually include components in addition to the antigens e.g. they typically include one or more pharmaceutical carrier(s) and/or excipient(s). As described below, adjuvants may also be included. A thorough discussion of such components is available in reference 63.

Compositions will generally be in aqueous form.

The composition may include preservatives such as thiomersal or 2-phenoxyethanol. It is preferred, however, that the vaccine should be substantially free from (*i.e.* less than 5µg/ml) mercurial material *e.g.* thiomersal-free [29,64]. Vaccines containing no mercury are more preferred. Preservative-free vaccines are particularly preferred. α-tocopherol succinate can be included as an alternative to mercurial compounds [29].

To control tonicity, it is preferred to include a physiological salt, such as a sodium salt. Sodium chloride (NaCl) is preferred, which may be present at between 1 and 20 mg/ml. Other salts that may be present include potassium chloride, potassium dihydrogen phosphate, disodium phosphate dehydrate, magnesium chloride, calcium chloride, *etc.*

Compositions will generally have an osmolality of between 200 mOsm/kg and 400 mOsm/kg, preferably between 240-360 mOsm/kg, and will more preferably fall within the range of 290-310 mOsm/kg. Osmolality has previously been reported not to have an impact on pain caused by vaccination [65], but keeping osmolality in this range is nevertheless preferred.

Compositions may include one or more buffers. Typical buffers include: a phosphate buffer; a Tris buffer; a borate buffer; a succinate buffer; a histidine buffer (particularly with an aluminum hydroxide adjuvant); or a citrate buffer. Buffers will typically be included in the 5-20mM range.

The pH of a composition will generally be between 5.0 and 8.1, and more typically between 6.0 and 8.0 *e.g.* 6.5 and 7.5, or between 7.0 and 7.8. A process of the invention may therefore include a step of adjusting the pH of the bulk vaccine prior to packaging.

The composition is preferably sterile. The composition is preferably non-pyrogenic *e.g.* containing <1 EU (endotoxin unit, a standard measure) per dose, and preferably <0.1 EU per dose. The composition is preferably gluten free.

Compositions of the invention may include detergent *e.g.* a polyoxyethylene sorbitan ester surfactant (known as 'Tweens'), an octoxynol (such as octoxynol-9 (Triton X-100) or t-octylphenoxypolyethoxyethanol), a cetyl trimethyl ammonium bromide ('CTAB'), or sodium deoxycholate, particularly for a split or surface antigen vaccine. The detergent may be present only at trace amounts. Thus the vaccine may included less than 1mg/ml of each of octoxynol-10 and polysorbate 80. Other residual components in trace amounts could be antibiotics (*e.g.* neomycin, kanamycin, polymyxin B).

The composition may include material for a single immunisation, or may include material for multiple immunisations (*i.e.* a 'multidose' kit). The inclusion of a preservative is preferred in multidose arrangements. As an alternative (or in addition) to including a preservative in multidose compositions, the compositions may be contained in a container having an aseptic adaptor for removal of material.

Influenza vaccines are typically administered in a dosage volume of about 0.5ml, although a half dose (*i.e.* about 0.25ml) may be administered to children.

Compositions and kits are preferably stored at between 2°C and 8°C. They should not be frozen. They should ideally be kept out of direct light.

### Adjuvants

Compositions of the invention may advantageously include an adjuvant, which can function to enhance the immune responses (humoral and/or cellular) elicited in a patient who receives the composition. The use of adjuvants with influenza vaccines has been described before. In references 66 & 67, aluminum hydroxide was used, and in reference 68, a mixture of aluminum hydroxide and aluminum phosphate was used. Reference 69 also described the use of aluminum salt adjuvants. The FLUAD™ product from Chiron Vaccines includes an oil-in-water emulsion.

Adjuvants that can be used with the invention include, but are not limited to:
● A mineral-containing composition, including calcium salts and aluminum salts (or mixtures thereof). Calcium salts include calcium phosphate (*e.g.* the "CAP" particles disclosed in ref. 70). Aluminum salts include hydroxides, phosphates, sulfates, *etc.*, with the salts taking any suitable form (*e.g.* gel, crystalline, amorphous, *etc.*). Adsorption to these salts is preferred. The mineral containing compositions may also be formulated as a particle of metal salt [71]. Aluminum salt adjuvants are described in more detail below.
● Oil-in-water emulsions (see in more detail below).
● Saponins [chapter 22 of ref. 97], which are a heterologous group of sterol glycosides and triterpenoid glycosides that are found in the bark, leaves, stems, roots and even flowers of a wide range of plant species. Saponin from the bark of the *Quillaia saponaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs. QS21 is marketed as Stimulon™. Saponin compositions have been purified using HPLC and RP-HPLC. Specific purified fractions using these techniques have been identified, including QS7, QS17, QS18, QS21, QH-A, QH-B and QH-C. Preferably, the saponin is QS21. A method of production of QS21 is disclosed in ref. 72. Saponin formulations may also comprise a sterol, such as cholesterol [73]. Combinations of saponins and cholesterols can be used to form unique particles called immunostimulating complexs (ISCOMs) [chapter 23 of ref. 97]. ISCOMs typically also include a phospholipid such as phosphatidylethanolamine or phosphatidylcholine. Any known saponin can be used in ISCOMs. Preferably, the ISCOM includes one or more of QuilA, QHA & QHC. ISCOMs are further described in refs. 73-75. Optionally, the ISCOMS may be devoid of additional detergent [76]. A review of the development of saponin based adjuvants can be found in refs. 77 & 78.
● Fatty adjuvants (see in more detail below).
● Bacterial ADP-ribosylating toxins (*e.g.* the *E.coli* heat labile enterotoxin "LT", cholera toxin "CT", or pertussis toxin "PT") and detoxified derivatives thereof, such as the mutant toxins known as LT-K63 and LT-R72 [79]. The use of detoxified ADP-ribosylating toxins as mucosal adjuvants is described in ref. 80 and as parenteral adjuvants in ref. 81.
● Bioadhesives and mucoadhesives, such as esterified hyaluronic acid microspheres [82] or chitosan and its derivatives [83].
● Cytokine-inducing agents (see in more detail below).
● Microparticles (*i.e.* a particle of ~100nm to ~150µm in diameter, more preferably ~200nm to ~30µm in diameter, or ~500nm to ~10µm in diameter) formed from materials that are biodegradable and non-toxic (*e.g.* a poly(α-hydroxy acid), a polyhydroxybutyric acid, a polyorthoester, a polyanhydride, a polycaprolactone, *etc.),* with poly(lactide-co-glycolide) being preferred, optionally treated to have a negatively-charged surface (*e.g.* with SDS) or a positively-charged surface (*e.g.* with a cationic detergent, such as CTAB).
● Liposomes (Chapters 13 & 14 of ref. 97). Examples of liposome formulations suitable for use as adjuvants are described in refs. 84-86.
● Polyoxyethylene ethers and polyoxyethylene esters [87]. Such formulations further include polyoxyethylene sorbitan ester surfactants in combination with an octoxynol [88] as well as polyoxyethylene alkyl ethers or ester surfactants in combination with at least one additional non-ionic surfactant such as an octoxynol [89]. Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.
● Muramyl peptides, such as N-acetylmuramyl-L-threonyl-D-isoglutamine ("thr-MDP"), N-acetyl-normuramyl-L-alanyl-D-isoglutamine (nor-MDP), N-acetylglucsaminyl-N-acetylmuramyl-L-Al-D-isoglu-L-Ala-dipalmitoxy propylamide ("DTP-DPP", or "Theramide™), N-acetylmuramyl-L-alanyl-D-isoglutaminyl-L-alanine-2-(1'-2'dipalmitoyl-sn-glycero-3-hydroxyphosphoryloxy)-ethylamine ("MTP-PE").
● An outer membrane protein proteosome preparation prepared from a first Gram-negative bacterium in combination with a liposaccharide preparation derived from a second Gram-negative bacterium, wherein the outer membrane protein proteosome and liposaccharide preparations form a stable non-covalent adjuvant complex. Such complexes include "IVX-908", a complex comprised of *Neisseria meningitidis* outer membrane and lipopolysaccharides. They have been used as adjuvants for influenza vaccines [90].
● Methyl inosine 5'-monophosphate ("MIMP") [91].
● A polyhydroxlated pyrrolizidine compound [92], such as one having formula: 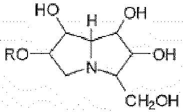 where R is selected from the group comprising hydrogen, straight or branched, unsubstituted or substituted, saturated or unsaturated acyl, alkyl (*e.g.* cycloalkyl), alkenyl, alkynyl and aryl groups, or a pharmaceutically acceptable salt or derivative thereof. Examples include, but are not limited to: casuarine, casuarine-6-α-D-glucopyranose, 3-*epi*-casuarine, 7-*epi*-casuarine, 3,7-die*pi*-casuarine, *etc.*
● A gamma inulin [93] or derivative thereof, such as algammulin.

These and other adjuvant-active substances are discussed in more detail in references 97 & 98.

Compositions may include two or more of said adjuvants. For example, they may advantageously include both an oil-in-water emulsion and a cytokine-inducing agent, as this combination improves the cytokine responses elicited by influenza vaccines, such as the interferon-γ response, with the improvement being much greater than seen when either the emulsion or the agent is used on its own.

Antigens and adjuvants in a composition will typically be in admixture.

### Oil-in-water emulsion adjuvants

Oil-in-water emulsions have been found to be particularly suitable for use in adjuvanting influenza virus vaccines. Various such emulsions are known, and they typically include at least one oil and at least one surfactant, with the oil(s) and surfactant(s) being biodegradable (metabolisable) and biocompatible. The oil droplets in the emulsion are generally less than 5µm in diameter, and may even have a sub-micron diameter, with these small sizes being achieved with a microfluidiser to provide stable emulsions. Droplets with a size less than 220nm are preferred as they can be subjected to filter sterilization.

The invention can be used with oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used e.g. obtained from the jojoba bean. Seed oils include safflower oil, cottonseed oil, sunflower seed oil, sesame seed oil, *etc.* In the grain group, corn oil is the most readily available, but the oil of other cereal grains such as wheat, oats, rye, rice, teff, triticale, *etc.* may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is particularly preferred herein. Squalane, the saturated analog to squalene, is also a preferred oil. Fish oils, including squalene and squalane, are readily available from commercial sources or may be obtained by methods known in the art. Other preferred oils are the tocopherols (see below). Mixtures of oils can be used.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); nonylphenol ethoxylates, such as the Tergitol™ NP series; polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Non-ionic surfactants are preferred. Preferred surfactants for including in the emulsion are Tween 80 (polyoxyethylene sorbitan monooleate), Span 85 (sorbitan trioleate), lecithin and Triton X-100.

Mixtures of surfactants can be used *e.g.* Tween 80/Span 85 mixtures. A combination of a polyoxyethylene sorbitan ester such as polyoxyethylene sorbitan monooleate (Tween 80) and an octoxynol such as t-octylphenoxypolyethoxyethanol (Triton X-100) is also suitable. Another useful combination comprises laureth 9 plus a polyoxyethylene sorbitan ester and/or an octoxynol.

Preferred amounts of surfactants (% by weight) are: polyoxyethylene sorbitan esters (such as Tween 80) 0.01 to 1%, in particular about 0.1 %; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to 1 % or about 0.5%.

Specific oil-in-water emulsion adjuvants useful with the invention include, but are not limited to:
● A submicron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [94-96], as described in more detail in Chapter 10 of ref. 97 and chapter 12 of ref. 98. The MF59 emulsion advantageously includes citrate ions *e.g.* 10mM sodium citrate buffer.
● An emulsion of squalene, a tocopherol, and Tween 80. The emulsion may include phosphate buffered saline. It may also include Span 85 (*e.g.* at 1%) and/or lecithin. These emulsions may have from 2 to 10% squalene, from 2 to 10% tocopherol and from 0.3 to 3% Tween 80, and the weight ratio of squalene:tocopherol is preferably ≤1 as this provides a more stable emulsion. Squalene and Tween 80 may be present volume ratio of about 5:2. One such emulsion can be made by dissolving Tween 80 in PBS to give a 2% solution, then mixing 90ml of this solution with a mixture of (5g of DL-α-tocopherol and 5ml squalene), then microfluidising the mixture. The resulting emulsion may have submicron oil droplets *e.g.* with an average diameter of between 100 and 250nm, preferably about 180nm.

● An emulsion of squalene, a tocopherol, and a Triton detergent (*e.g.* Triton X-100). The emulsion may also include a 3d-MPL (see below). The emulsion may contain a phosphate buffer.
● An emulsion comprising a polysorbate (*e.g.* polysorbate 80), a Triton detergent (*e.g.* Triton X-100) and a tocopherol (*e.g.* an α-tocopherol succinate). The emulsion may include these three components at a mass ratio of about 75:11:10 (*e.g.* 750µg/ml polysorbate 80, 110µg/ml Triton X-100 and 100µg/ml α-tocopherol succinate), and these concentrations should include any contribution of these components from antigens. The emulsion may also include squalene. The emulsion may also include a 3d-MPL (see below). The aqueous phase may contain a phosphate buffer.
● An emulsion of squalane, polysorbate 80 and poloxamer 401 ("Pluronic™ L121"). The emulsion can be formulated in phosphate buffered saline, pH 7.4. This emulsion is a useful delivery vehicle for muramyl dipeptides, and has been used with threonyl-MDP in the "SAF-1" adjuvant [99] (0.05-1% Thr-MDP, 5% squalane, 2.5% Pluronic L121 and 0.2% polysorbate 80). It can also be used without the Thr-MDP, as in the "AF" adjuvant [100] (5% squalane, 1.25% Pluronic L121 and 0.2% polysorbate 80). Microfluidisation is preferred.
● An emulsion comprising squalene, an aqueous solvent, a polyoxyethylene alkyl ether hydrophilic nonionic surfactant (*e.g.* polyoxyethylene (12) cetostearyl ether) and a hydrophobic nonionic surfactant (*e.g.* a sorbitan ester or mannide ester, such as sorbitan monoleate or 'Span 80'). The emulsion is preferably thermoreversible and/or has at least 90% of the oil droplets (by volume) with a size less than 200 nm [101]. The emulsion may also include one or more of: alditol; a cryoprotective agent (*e.g.* a sugar, such as dodecylmaltoside and/or sucrose); and/or an alkylpolyglycoside. Such emulsions may be lyophilized.
● An emulsion of squalene, poloxamer 105 and Abil-Care [102]. The final concentration (weight) of these components in adjuvanted vaccines are 5% squalene, 4% poloxamer 105 (pluronic polyol) and 2% Abil-Care 85 (Bis-PEG/PPG-16/16 PEG/PPG-16/16 dimethicone; caprylic/capric triglyceride).
● An emulsion having from 0.5-50% of an oil, 0.1-10% of a phospholipid, and 0.05-5% of a non-ionic surfactant. As described in reference 103, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
● A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 104, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
● An emulsion in which a saponin (*e.g.* QuilA or QS21) and a sterol (*e.g.* a cholesterol) are associated as helical micelles [105].
● An emulsion comprising a mineral oil, a non-ionic lipophilic ethoxylated fatty alcohol, and a non-ionic hydrophilic surfactant (*e.g.* an ethoxylated fatty alcohol and/or polyoxyethylene-polyoxypropylene block copolymer) [106].
● An emulsion comprising a mineral oil, a non-ionic hydrophilic ethoxylated fatty alcohol, and a non-ionic lipophilic surfactant (*e.g.* an ethoxylated fatty alcohol and/or polyoxyethylene-polyoxypropylene block copolymer) [106].

The emulsions may be mixed with antigen extemporaneously, at the time of delivery. Thus the adjuvant and antigen may be kept separately in a packaged or distributed vaccine, ready for final formulation at the time of use. The antigen will generally be in an aqueous form, such that the vaccine is finally prepared by mixing two liquids. The volume ratio of the two liquids for mixing can vary (*e.g.* between 5:1 and 1:5) but is generally about 1:1.

After the antigen and adjuvant have been mixed, haemagglutinin antigen will generally remain in aqueous solution but may distribute itself around the oil/water interface. In general, little if any haemagglutinin will enter the oil phase of the emulsion.

Where a composition includes a tocopherol, any of the α, β, γ, δ, ε or ξ tocopherols can be used, but α-tocopherols are preferred. The tocopherol can take several forms *e.g.* different salts and/or isomers. Salts include organic salts, such as succinate, acetate, nicotinate, *etc.* D-α-tocopherol and DL-α-tocopherol can both be used. Tocopherols are advantageously included in vaccines for use in elderly patients (*e.g.* aged 60 years or older) because vitamin E has been reported to have a positive effect on the immune response in this patient group [107]. They also have antioxidant properties that may help to stabilize the emulsions [108]. A preferred α-tocopherol is DL-α-tocopherol, and the preferred salt of this tocopherol is the succinate. The succinate salt has been found to cooperate with TNF-related ligands *in vivo.* Moreover, α-tocopherol succinate is known to be compatible with influenza vaccines and to be a useful preservative as an alternative to mercurial compounds [29].

### Cytokine-inducing agents

Cytokine-inducing agents for inclusion in compositions of the invention are able, when administered to a patient, to elicit the immune system to release cytokines, including interferons and interleukins. Cytokine responses are known to be involved in the early and decisive stages of host defense against influenza infection [109]. Preferred agents can elicit the release of one or more of: interferon-γ; interleukin-1; interleukin-2; interleukin-12; TNF-α; TNF-β; and GM-CSF. Preferred agents elicit the release of cytokines associated with a Th1-type immune response *e.g.* interferon-γ, TNF-α, interleukin-2. Stimulation of both interferon-γ and interleukin-2 is preferred.

As a result of receiving a composition of the invention, therefore, a patient will have T cells that, when stimulated with an influenza antigen, will release the desired cytokine(s) in an antigen-specific manner. For example, T cells purified form their blood will release γ-interferon when exposed *in vitro* to influenza virus haemagglutinin. Methods for measuring such responses in peripheral blood mononuclear cells (PBMC) are known in the art, and include ELISA, ELISPOT, flow-cytometry and real-time PCR. For example, reference 110 reports a study in which antigen-specific T cell-mediated immune responses against tetanus toxoid, specifically γ-interferon responses, were monitored, and found that ELISPOT was the most sensitive method to discriminate antigen-specific TT-induced responses from spontaneous responses, but that intracytoplasmic cytokine detection by flow cytometry was the most efficient method to detect re-stimulating effects.

Suitable cytokine-inducing agents include, but are not limited to:
● An immunostimulatory oligonucleotide, such as one containing a CpG motif (a dinucleotide sequence containing an unmethylated cytosine linked by a phosphate bond to a guanosine), or a double-stranded RNA, or an oligonucleotide containing a palindromic sequence, or an oligonucleotide containing a poly(dG) sequence.
● 3-O-deacylated monophosphoryl lipid A ('3dMPL', also known as 'MPL™') [111-114].
● An imidazoquinoline compound, such as Imiquimod ("R-837") [115,116], Resiquimod ("R-848") [117], and their analogs; and salts thereof (e.g. the hydrochloride salts). Further details about immunostimulatory imidazoquinolines can be found in references 118 to 122.
● A thiosemicarbazone compound, such as those disclosed in reference 123. Methods of formulating, manufacturing, and screening for active compounds are also described in reference 123. The thiosemicarbazones are particularly effective in the stimulation of human peripheral blood mononuclear cells for the production of cytokines, such as TNF-α.
● A tryptanthrin compound, such as those disclosed in reference 124. Methods of formulating, manufacturing, and screening for active compounds are also described in reference 124. The thiosemicarbazones are particularly effective in the stimulation of human peripheral blood mononuclear cells for the production of cytokines, such as TNF-α.
● A nucleoside analog, such as: (a) Isatorabine (ANA-245; 7-thia-8-oxoguanosine): 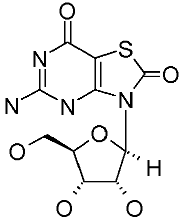 and prodrugs thereof; (b) ANA975; (c) ANA-025-1; (d) ANA380; (e) the compounds disclosed in references 125 to 127; (f) a compound having the formula: 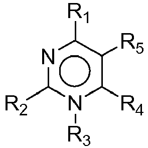 wherein:
   R₁ and R₂ are each independently H, halo, -NRₐR_{b}, -OH, C₁₋₆ alkoxy, substituted C₁₋₆ alkoxy, heterocyclyl, substituted heterocyclyl, C₆₋₁₀ aryl, substituted C₆₋₁₀ aryl, C₁₋₆ alkyl, or substituted C₁₋₆ alkyl;
   R₃ is absent, H, C₁₋₆ alkyl, substituted C₁₋₆ alkyl, C₆₋₁₀ aryl, substituted C₆₋₁₀ aryl, heterocyclyl, or substituted heterocyclyl;
   R₄ and R₅ are each independently H, halo, heterocyclyl, substituted heterocyclyl, -C(O)-R_{d}, C₁₋₆ alkyl, substituted C₁₋₆ alkyl, or bound together to form a 5 membered ring as in R₄₋₅: 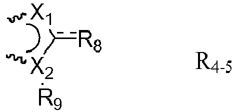 the binding being achieved at the bonds indicated by a 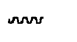
   X₁ and X₂ are each independently N, C, O, or S;
   R₈ is H, halo, -OH, C₁₋₆ alkyl, C₂₋₆ alkenyl, C₂₋₆ alkynyl, -OH, -NRₐR_{b}, -(CH₂)ₙ-O-R_{c}, -O-(C₁₋₆ alkyl), -S(O)ₚRₑ, or -C(O)-R_{d};
   R₉ is H, C₁₋₆ alkyl, substituted C₁₋₆ alkyl, heterocyclyl, substituted heterocyclyl or R₉ₐ, wherein R₉ₐ is: 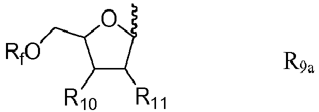 the binding being achieved at the bond indicated by a 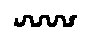
   R₁₀ and R₁₁ are each independently H, halo, C₁₋₆ alkoxy, substituted C₁₋₆ alkoxy, - NRₐR_{b}, or -OH;
   each Rₐ and R_{b} is independently H, C₁₋₆ alkyl, substituted C₁₋₆ alkyl, -C(O)R_{d}, C₆₋₁₀ aryl;
   each R_{c} is independently H, phosphate, diphosphate, triphosphate, C₁₋₆ alkyl, or substituted C₁₋₆ alkyl;
   each R_{d} is independently H, halo, C₁₋₆ alkyl, substituted C₁₋₆ alkyl, C₁₋₆ alkoxy, substituted C₁₋₆ alkoxy, -NH₂, -NH(C₁₋₆ alkyl), -NH(substituted C₁₋₆ alkyl), -N(C₁₋₆ alkyl)₂, -N(substituted C₁₋₆ alkyl)₂, C₆₋₁₀ aryl, or heterocyclyl;
   each Rₑ is independently H, C₁₋₆ alkyl, substituted C₁₋₆ alkyl, C₆₋₁₀ aryl, substituted C₆₋₁₀ aryl, heterocyclyl, or substituted heterocyclyl;
   each R_{f} is independently H, C₁₋₆ alkyl, substituted C₁₋₆ alkyl, -C(O)R_{d}, phosphate, diphosphate, or triphosphate;
   each n is independently 0, 1, 2, or 3;
   each p is independently 0, 1, or 2; or
   or (g) a pharmaceutically acceptable salt of any of (a) to (f), a tautomer of any of (a) to (f), or a pharmaceutically acceptable salt of the tautomer.
● Loxoribine (7-allyl-8-oxoguanosine) [128].
● Compounds disclosed in reference 129, including: Acylpiperazine compounds, Indoledione compounds, Tetrahydraisoquinoline (THIQ) compounds, Benzocyclodione compounds, Aminoazavinyl compounds, Aminobenzimidazole quinolinone (ABIQ) compounds [130,131], Hydrapthalamide compounds, Benzophenone compounds, Isoxazole compounds, Sterol compounds, Quinazilinone compounds, Pyrrole compounds [132], Anthraquinone compounds, Quinoxaline compounds, Triazine compounds, Pyrazalopyrimidine compounds, and Benzazole compounds [133].
● A polyoxidonium polymer [134,135] or other N-oxidized polyethylene-piperazine derivative.
● Compounds disclosed in reference 136.
● An aminoalkyl glucosaminide phosphate derivative, such as RC-529 [137,138].
● A phosphazene, such as poly[di(carboxylatophenoxy)phosphazene] ("PCPP") as described, for example, in references 139 and 140.
● A CDld ligand, such as an α-glycosylceramide [141-148] (*e.g.* α-galactosylceramide), phytosphingosine-containing α-glycosylceramides, OCH, KRN7000 [(2S,3S,4R)-1-O-(α-D-galactopyranosyl)-2-(N-hexacosanoylamino)-1,3,4-octadecanetriol], CRONY-101, 3"-O-sulfo-galactosylceramide, *etc.*
● Small molecule immunopotentiators (SMIPs) such as:
   N2-methyl-1-(2-methylpropyl)-1H-imidazo[4,5-c]quinoline-2,4-diamine
   N2,N2-dimethyl-1-(2-methylpropyl)-1H-imidazo[4,5-c]quinoline-2,4-diamine
   N2-ethyl-N2-methyl-1-(2-methylpropyl)-1H-imidazo[4,5-c]quinoline-2,4-diamine
   N2-methyl-1-(2-methylpropyl)-N2-propyl-1H-imidazo[4,5-c]quinoline-2,4-diamine
   1-(2-methylpropyl)-N2-propyl-1H-imidazo[4,5-c]quinoline-2,4-diamine
   N2-butyl-1-(2-methylpropyl)-1H-imidazo[4,5-c]quinoline-2,4-diamine
   N2-butyl-N2-methyl-1-(2-methylpropyl)-1H-imidazo[4,5-c]quinoline-2,4-diamine
   N2-methyl-1-(2-methylpropyl)-N2-pentyl-1H-imidazo[4,5-c]quinoline-2,4-diamine
   N2-methyl-1-(2-methylpropyl)-N2-prop-2-enyl-1H-imidazo[4,5-c]quinoline-2,4-diamine
   1-(2-methylpropyl)-2-[(phenylmethyl)thio]-1H-imidazo[4,5-c]quinolin-4-amine
   1-(2-methylpropyl)-2-(propylthio)-1H-imidazo[4,5-c]quinolin-4-amine
   2-[[4-amino-1-(2-methylpropyl)-1H-imidazo[4,5-c]quinolin-2-yl](methyl)amino]ethanol 2-[[4-amino-1-(2-methylpropyl)-1H-imidazo[4,5-c]quinolin-2-yl](methyl)amino]ethyl acetate 4-amino-1-(2-methylpropyl)-1,3-dihydro-2H-imidazo[4,5-c]quinolin-2-one N2-butyl-1-(2-methylpropyl)-N4,N4-bis(phenylmethyl)-1H-imidazo[4,5-c]quinoline-2,4-diamine N2-butyl-N2-methyl-1-(2-methylpropyl)-N4,N4-bis(phenylmethyl)-1H-imidazo[4,5-c]quinoline-2,4-diamine
   N2-methyl-1-(2-methylpropyl)-N4,N4-bis(phenylmethyl)-1H-imidazo[4,5-c]quinoline-2,4-diamine
   N2,N2-dimethyl-1-(2-methylpropyl)-N4,N4-bis(phenylmethyl)-1H-imidazo[4,5-c]quinoline-2,4-diamine
   1-{4-amino-2-[methyl(propyl)amino]-1H-imidazo[4,5-c]quinolin-1-yl}-2-methylpropan-2-ol 1-[4-amino-2-(propylamino)-1H-imidazo[4,5-c]quinolin-1-yl]-2-methylpropan-2-ol N4,N4-dibenzyl-1-(2-methoxy-2-methylpropyl)-N2-propyl-1H-imidazo[4,5-c]quinoline-2,4-diamine.

The cytokine-inducing agents for use in the present invention may be modulators and/or agonists of Toll-Like Receptors (TLR). For example, they may be agonists of one or more of the human TLR1, TLR2, TLR3, TLR4, TLR7, TLR8, and/or TLR9 proteins. Preferred agents are agonists of TLR7 (*e.g.* imidazoquinolines) and/or TLR9 (*e.g.* CpG oligonucleotides). These agents are useful for activating innate immunity pathways.

The cytokine-inducing agent can be added to the composition at various stages during its production. For example, it may be within an antigen composition, and this mixture can then be added to an oil-in-water emulsion. As an alternative, it may be within an oil-in-water emulsion, in which case the agent can either be added to the emulsion components before emulsification, or it can be added to the emulsion after emulsification. Similarly, the agent may be coacervated within the emulsion droplets. The location and distribution of the cytokine-inducing agent within the final composition will depend on its hydrophilic/lipophilic properties *e.g.* the agent can be located in the aqueous phase, in the oil phase, and/or at the oil-water interface.

The cytokine-inducing agent can be conjugated to a separate agent, such as an antigen (*e.g.* CRM197). A general review of conjugation techniques for small molecules is provided in ref. 149. As an alternative, the adjuvants may be non-covalently associated with additional agents, such as by way of hydrophobic or ionic interactions.

Two preferred cytokine-inducing agents are (a) immunostimulatory oligonucleotides and (b) 3dMPL.

Immunostimulatory oligonucleotides can include nucleotide modifications/analogs such as phosphorothioate modifications and can be double-stranded or (except for RNA) single-stranded. References 150, 151 and 152 disclose possible analog substitutions *e.g.* replacement of guanosine with 2'-deoxy-7-deazaguanosine. The adjuvant effect of CpG oligonucleotides is further discussed in refs. 153-158. A CpG sequence may be directed to TLR9, such as the motif GTCGTT or TTCGTT [159]. The CpG sequence may be specific for inducing a Th1 immune response, such as a CpG-A ODN (oligodeoxynucleotide), or it may be more specific for inducing a B cell response, such a CpG-B ODN. CpG-A and CpG-B ODNs are discussed in refs. 160-162. Preferably, the CpG is a CpG-A ODN. Preferably, the CpG oligonucleotide is constructed so that the 5' end is accessible for receptor recognition. Optionally, two CpG oligonucleotide sequences may be attached at their 3' ends to form "immunomers". See, for example, references 159 & 163-165. A useful CpG adjuvant is CpG7909, also known as ProMune™ (Coley Pharmaceutical Group, Inc.).

As an alternative, or in addition, to using CpG sequences, TpG sequences can be used [166]. These oligonucleotides may be free from unmethylated CpG motifs.

The immunostimulatory oligonucleotide may be pyrimidine-rich. For example, it may comprise more than one consecutive thymidine nucleotide (*e.g.* TTTT, as disclosed in ref. 166), and/or it may have a nucleotide composition with >25% thymidine (*e.g.* >35%, >40%, >50%, >60%, >80%, *etc.).* For example, it may comprise more than one consecutive cytosine nucleotide (e.g. CCCC, as disclosed in ref. 166), and/or it may have a nucleotide composition with >25% cytosine (*e.g.* >35%, >40%, >50%, >60%, >80%, *etc*.). These oligonucleotides may be free from unmethylated CpG motifs.

Immunostimulatory oligonucleotides will typically comprise at least 20 nucleotides. They may comprise fewer than 100 nucleotides.

A particularly useful adjuvant based around immunostimulatory oligonucleotides is known as IC31™ [167]. Thus an adjuvant used with the invention may comprise a mixture of (i) an oligonucleotide (*e.g.* between 15-40 nucleotides) including at least one (and preferably multiple) CpI motifs, and (ii) a polycationic polymer, such as an oligopeptide (e.g. between 5-20 amino acids) including at least one (and preferably multiple) Lys-Arg-Lys tripeptide sequence(s). The oligonucleotide may be a deoxynucleotide comprising 26-mer sequence 5'-(IC)₁₃-3' (SEQ ID NO: 1). The polycationic polymer may be a peptide comprising 11-mer amino acid sequence KLKLLLLLKLK (SEQ ID NO: 2).

3dMPL (also known as 3 de-O-acylated monophosphoryl lipid A or 3-O-desacyl-4'-monophosphoryl lipid A) is an adjuvant in which position 3 of the reducing end glucosamine in monophosphoryl lipid A has been de-acylated. 3dMPL has been prepared from a heptoseless mutant of *Salmonella minnesota*, and is chemically similar to lipid A but lacks an acid-labile phosphoryl group and a base-labile acyl group. It activates cells of the monocyte/macrophage lineage and stimulates release of several cytokines, including IL-1, IL-12, TNF-α and GM-CSF (see also ref. 168). Preparation of 3dMPL was originally described in reference 169.

3dMPL can take the form of a mixture of related molecules, varying by their acylation (e.g. having 3, 4, 5 or 6 acyl chains, which may be of different lengths). The two glucosamine (also known as 2-deoxy-2-amino-glucose) monosaccharides are N-acylated at their 2-position carbons (*i.e.* at positions 2 and 2'), and there is also O-acylation at the 3' position. The group attached to carbon 2 has formula -NH-CO-CH₂-CR¹R^{1'}. The group attached to carbon 2' has formula -NH-CO-CH₂-CR²R^{2'}. The group attached to carbon 3' has formula -O-CO-CH₂-CR³R^{3'}. A representative structure is:

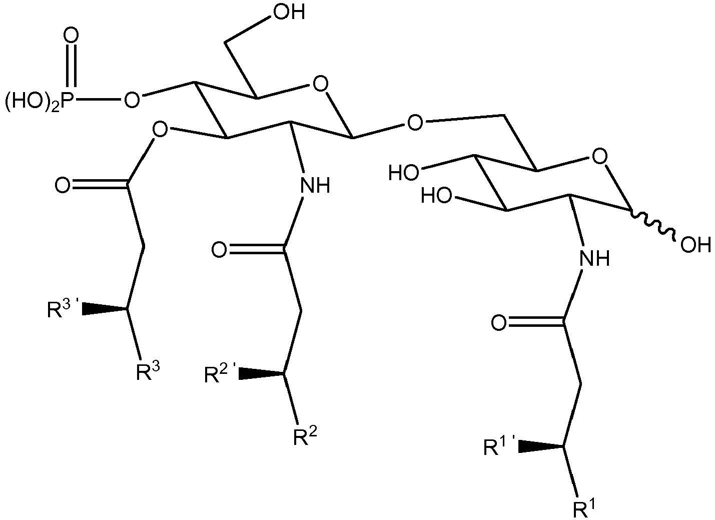

Groups R¹, R² and R³ are each independently -(CH₂)ₙ-CH₃. The value of *n* is preferably between 8 and 16, more preferably between 9 and 12, and is most preferably 10.

Groups R^{1'}, R^{2'} and R^{3'} can each independently be: (a) -H; (b) -OH; or (c) -O-CO-R⁴,where R⁴ is either -H or -(CH₂)ₘ-CH₃, wherein the value of *m* is preferably between 8 and 16, and is more preferably 10, 12 or 14. At the 2 position, *m* is preferably 14. At the 2' position, *m* is preferably 10. At the 3' position, *m* is preferably 12. Groups R^{1'}, R^{2'} and R^{3'} are thus preferably -O-acyl groups from dodecanoic acid, tetradecanoic acid or hexadecanoic acid.

When all of R^{1'}, R^{2'} and R^{3'} are -H then the 3dMPL has only 3 acyl chains (one on each of positions 2, 2' and 3'). When only two of R^{1'}, R^{2'} and R^{3'} are -H then the 3dMPL can have 4 acyl chains. When only one of R^{1'}, R^{2'} and R^{3'} is -H then the 3dMPL can have 5 acyl chains. When none of R^{1'}, R^{2'} and R^{3'} is -H then the 3dMPL can have 6 acyl chains. The 3dMPL adjuvant used according to the invention can be a mixture of these forms, with from 3 to 6 acyl chains, but it is preferred to include 3dMPL with 6 acyl chains in the mixture, and in particular to ensure that the hexaacyl chain form makes up at least 10% by weight of the total 3dMPL e.g. ≥20%, ≥30%, ≥40%, ≥50% or more. 3dMPL with 6 acyl chains has been found to be the most adjuvant-active form.

Thus the most preferred form of 3dMPL for inclusion in compositions of the invention has formula (IV):

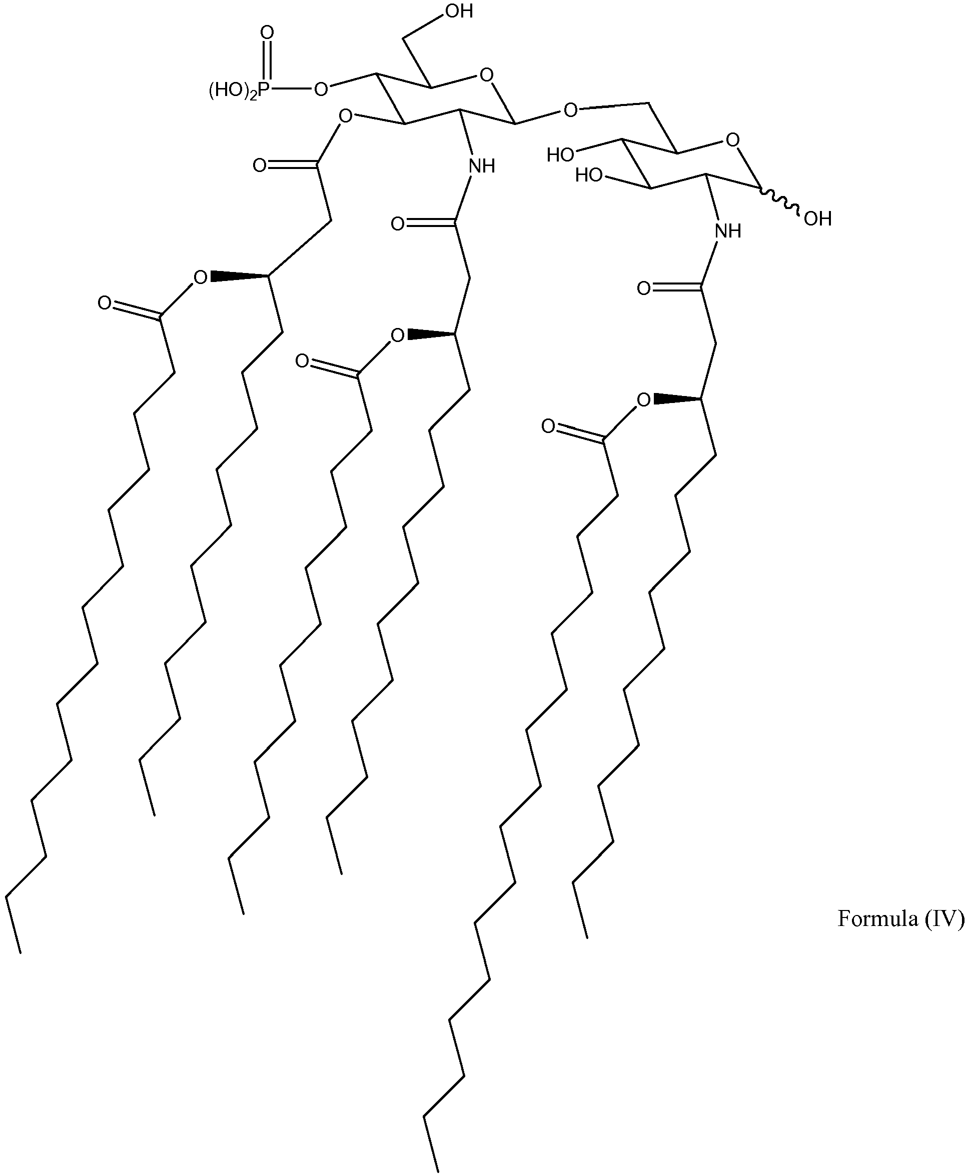

Where 3dMPL is used in the form of a mixture then references to amounts or concentrations of 3dMPL in compositions of the invention refer to the combined 3dMPL species in the mixture.

In aqueous conditions, 3dMPL can form micellar aggregates or particles with different sizes *e.g.* with a diameter <150nm or >500nm. Either or both of these can be used with the invention, and the better particles can be selected by routine assay. Smaller particles (*e.g.* small enough to give a clear aqueous suspension of 3dMPL) are preferred for use according to the invention because of their superior activity [170]. Preferred particles have a mean diameter less than 220nm, more preferably less than 200nm or less than 150nm or less than 120nm, and can even have a mean diameter less than 100nm. In most cases, however, the mean diameter will not be lower than 50nm. These particles are small enough to be suitable for filter sterilization. Particle diameter can be assessed by the routine technique of dynamic light scattering, which reveals a mean particle diameter. Where a particle is said to have a diameter of x nm, there will generally be a distribution of particles about this mean, but at least 50% by number (*e.g.* ≥60%, ≥70%, ≥80%, ≥90%, or more) of the particles will have a diameter within the range *x*±25%.

3dMPL can advantageously be used in combination with an oil-in-water emulsion. Substantially all of the 3dMPL may be located in the aqueous phase of the emulsion.

The 3dMPL can be used on its own, or in combination with one or more further compounds. For example, it is known to use 3dMPL in combination with the QS21 saponin [171] (including in an oil-in-water emulsion [172]), with an immunostimulatory oligonucleotide, with both QS21 and an immunostimulatory oligonucleotide, with aluminum phosphate [173], with aluminum hydroxide [174], or with both aluminum phosphate and aluminum hydroxide.

### Fatty adjuvants

Fatty adjuvants that can be used with the invention include the oil-in-water emulsions described above, and also include, for example:
● A compound of formula I, II or III, or a salt thereof: 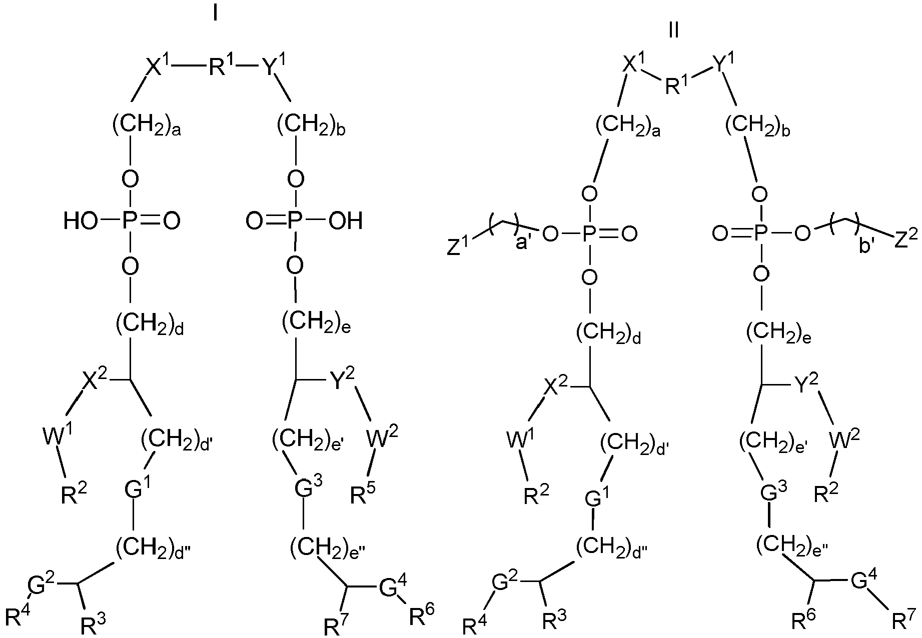 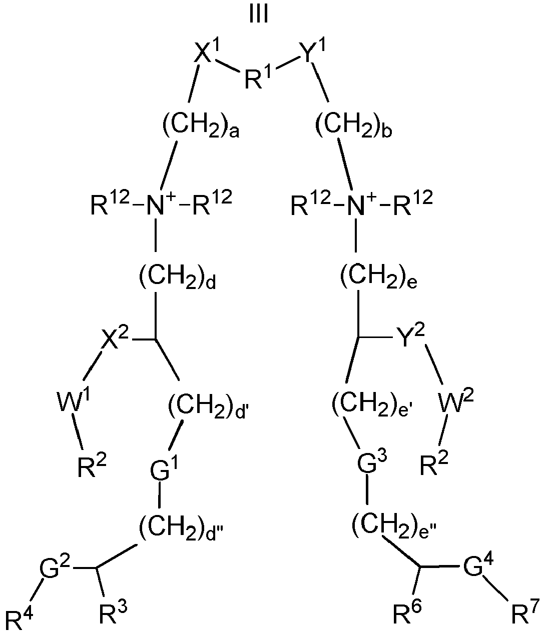 as defined in reference 175, such as 'ER 803058', 'ER 803732', 'ER 804053', ER 804058', 'ER 804059', 'ER 804442', 'ER 804680', 'ER 804764', ER 803022 or 'ER 804057' e.g.: 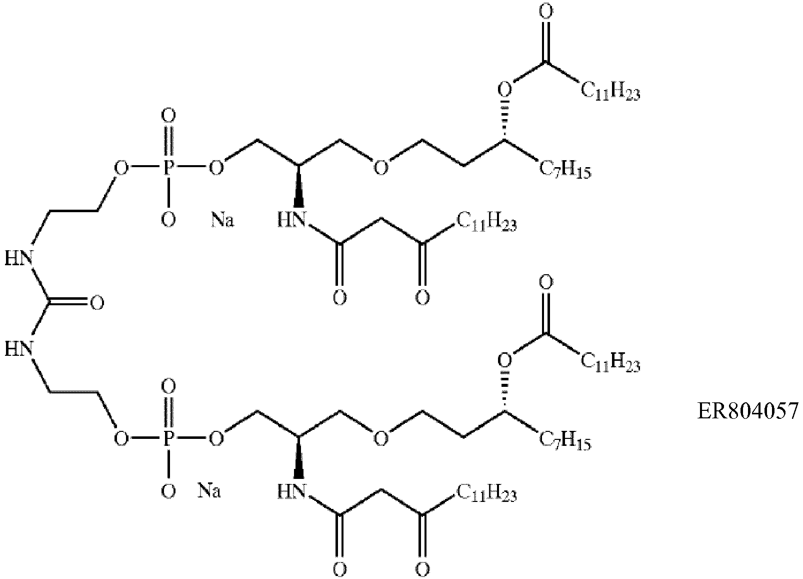 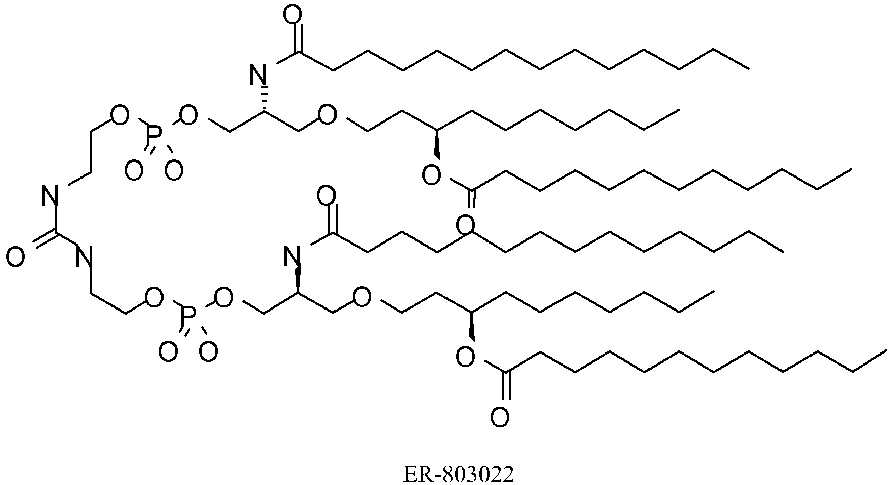
● Derivatives of lipid A from *Escherichia coli* such as OM-174 (described in refs. 176 & 177).
● A formulation of a cationic lipid and a (usually neutral) co-lipid, such as aminopropyl-dimethyl-myristoleyloxy-propanaminium bromide-diphytanoylphosphatidyl-ethanolamine ("Vaxfectin™") or aminopropyl-dimethyl-bis-dodecyloxy-propanaminium bromide-dioleoylphosphatidyl-ethanolamine ("GAP-DLRIE:DOPE"). Formulations containing (±)-N-(3-aminopropyl)-N,N-dimethyl-2,3-bis(syn-9-tetradeceneyloxy)-1-propanaminium salts are preferred [178].
● 3-O-deacylated monophosphoryl lipid A (see above).
● Compounds containing lipids linked to a phosphate-containing acyclic backbone, such as the TLR4 antagonist E5564 [179,180]:

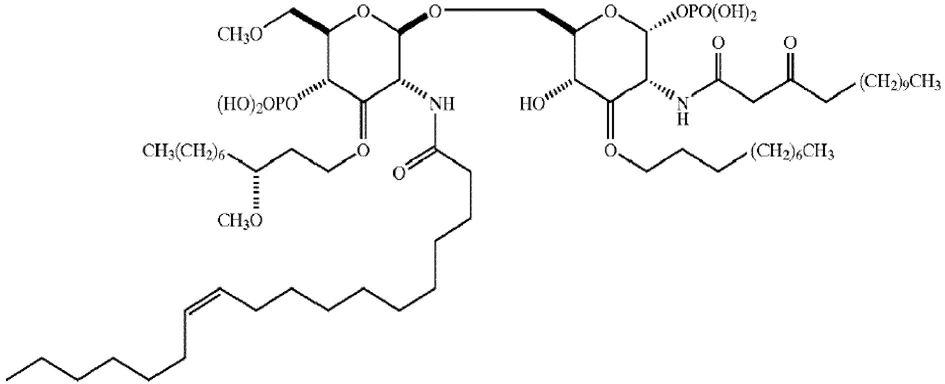

### Aluminum salt adjuvants

The adjuvants known as aluminum hydroxide and aluminum phosphate may be used. These names are conventional, but are used for convenience only, as neither is a precise description of the actual chemical compound which is present (e.g. see chapter 9 of reference 97181). The invention can use any of the "hydroxide" or "phosphate" adjuvants that are in general use as adjuvants.

The adjuvants known as "aluminium hydroxide" are typically aluminium oxyhydroxide salts, which are usually at least partially crystalline. Aluminium oxyhydroxide, which can be represented by the formula AlO(OH), can be distinguished from other aluminium compounds, such as aluminium hydroxide Al(OH)₃, by infrared (IR) spectroscopy, in particular by the presence of an adsorption band at 1070cm⁻¹ and a strong shoulder at 3090-3100cm⁻¹ [chapter 9 of ref. 97]. The degree of crystallinity of an aluminium hydroxide adjuvant is reflected by the width of the diffraction band at half height (WHH), with poorly-crystalline particles showing greater line broadening due to smaller crystallite sizes. The surface area increases as WHH increases, and adjuvants with higher WHH values have been seen to have greater capacity for antigen adsorption. A fibrous morphology (e.g. as seen in transmission electron micrographs) is typical for aluminium hydroxide adjuvants. The pI of aluminium hydroxide adjuvants is typically about 11 *i.e.* the adjuvant itself has a positive surface charge at physiological pH. Adsorptive capacities of between 1.8-2.6 mg protein per mg Al⁺⁺⁺ at pH 7.4 have been reported for aluminium hydroxide adjuvants.

The adjuvants known as "aluminium phosphate" are typically aluminium hydroxyphosphates, often also containing a small amount of sulfate (*i.e.* aluminium hydroxyphosphate sulfate). They may be obtained by precipitation, and the reaction conditions and concentrations during precipitation influence the degree of substitution of phosphate for hydroxyl in the salt. Hydroxyphosphates generally have a PO₄/Al molar ratio between 0.3 and 1.2. Hydroxyphosphates can be distinguished from strict AlPO₄ by the presence of hydroxyl groups. For example, an IR spectrum band at 3164cm⁻¹ (*e.g*. when heated to 200°C) indicates the presence of structural hydroxyls [ch.9 of ref. 97].

The PO₄/Al³⁺ molar ratio of an aluminium phosphate adjuvant will generally be between 0.3 and 1.2, preferably between 0.8 and 1.2, and more preferably 0.95±0.1. The aluminium phosphate will generally be amorphous, particularly for hydroxyphosphate salts. A typical adjuvant is amorphous aluminium hydroxyphosphate with PO₄/Al molar ratio between 0.84 and 0.92, included at 0.6mg Al³⁺/ml. The aluminium phosphate will generally be particulate (e.g. plate-like morphology as seen in transmission electron micrographs). Typical diameters of the particles are in the range 0.5-20µm (e.g. about 5-10µm) after any antigen adsorption. Adsorptive capacities of between 0.7-1.5 mg protein per mg Al⁺⁺⁺ at pH 7.4 have been reported for aluminium phosphate adjuvants.

The point of zero charge (PZC) of aluminium phosphate is inversely related to the degree of substitution of phosphate for hydroxyl, and this degree of substitution can vary depending on reaction conditions and concentration of reactants used for preparing the salt by precipitation. PZC is also altered by changing the concentration of free phosphate ions in solution (more phosphate = more acidic PZC) or by adding a buffer such as a histidine buffer (makes PZC more basic). Aluminium phosphates used according to the invention will generally have a PZC of between 4.0 and 7.0, more preferably between 5.0 and 6.5 *e.g.* about 5.7.

Suspensions of aluminium salts used to prepare compositions of the invention may contain a buffer (*e.g.* a phosphate or a histidine or a Tris buffer), but this is not always necessary. The suspensions are preferably sterile and pyrogen-free. A suspension may include free aqueous phosphate ions *e.g.* present at a concentration between 1.0 and 20 mM, preferably between 5 and 15 mM, and more preferably about 10 mM. The suspensions may also comprise sodium chloride.

The invention can use a mixture of both an aluminium hydroxide and an aluminium phosphate [68]. In this case there may be more aluminium phosphate than hydroxide *e.g.* a weight ratio of at least 2:1 *e.g.* ≥5:1, ≥6:1, ≥7:1, ≥8:1, ≥9:1, *etc.*

The concentration of Al⁺⁺⁺ in a composition for administration to a patient is preferably less than 10mg/ml *e.g.* ≤5 mg/ml, ≤4 mg/ml, ≤3 mg/ml, ≤2 mg/ml, ≤1 mg/ml, *etc.* A preferred range is between 0.3 and 1mg/ml. A maximum of <0.85mg/dose is preferred.

As well as including one or more aluminium salt adjuvants, the adjuvant component may include one or more further adjuvant or immunostimulating agents. Such additional components include, but are not limited to: a 3-O-deacylated monophosphoryl lipid A adjuvant ('3d-MPL'); and/or an oil-in-water emulsion. 3d-MPL has also been referred to as 3 de-O-acylated monophosphoryl lipid A or as 3-O-desacyl-4'-monophosphoryl lipid A. The name indicates that position 3 of the reducing end glucosamine in monophosphoryl lipid A is de-acylated. It has been prepared from a heptoseless mutant of *S.minnesota,* and is chemically similar to lipid A but lacks an acid-labile phosphoryl group and a base-labile acyl group. It activates cells of the monocyte/macrophage lineage and stimulates release of several cytokines, including IL-1, IL-12, TNF-α and GM-CSF. Preparation of 3d-MPL was originally described in reference 169, and the product has been manufactured and sold by Corixa Corporation under the name MPL™. Further details can be found in refs 111 to 114.

### Kits of the invention

Compositions of the invention may be prepared extemporaneously, at the time of delivery, particularly when an adjuvant is being used. Thus the invention provides kits including the various components ready for mixing. The kit allows the adjuvant and the antigen to be kept separately until the time of use. This arrangement can be useful when using an oil-in-water emulsion adjuvant.

The components are physically separate from each other within the kit, and this separation can be achieved in various ways. For instance, the two components may be in two separate containers, such as vials. The contents of the two vials can then be mixed e.g. by removing the contents of one vial and adding them to the other vial, or by separately removing the contents of both vials and mixing them in a third container.

In a preferred arrangement, one of the kit components is in a syringe and the other is in a container such as a vial. The syringe can be used (e.g. with a needle) to insert its contents into the second container for mixing, and the mixture can then be withdrawn into the syringe. The mixed contents of the syringe can then be administered to a patient, typically through a new sterile needle. Packing one component in a syringe eliminates the need for using a separate syringe for patient administration.

In another preferred arrangement, the two kit components are held together but separately in the same syringe *e.g.* a dual-chamber syringe, such as those disclosed in references 182-189 *etc.* When the syringe is actuated (*e.g.* during administration to a patient) then the contents of the two chambers are mixed. This arrangement avoids the need for a separate mixing step at the time of use.

The kit components will generally be in aqueous form. In some arrangements, a component (typically an antigen component rather than an adjuvant component) is in dry form (*e.g.* in a lyophilised form), with the other component being in aqueous form. The two components can be mixed in order to reactivate the dry component and give an aqueous composition for administration to a patient. A lyophilised component will typically be located within a vial rather than a syringe. Dried components may include stabilizers such as lactose, sucrose or mannitol, as well as mixtures thereof *e.g.* lactose/sucrose mixtures, sucrose/mannitol mixtures, *etc.* One possible arrangement uses an aqueous adjuvant component in a pre-filled syringe and a lyophilised antigen component in a vial.

### Packaging of compositions or kit components

Suitable containers for compositions of the invention (or kit components) include vials, syringes (*e.g.* disposable syringes), nasal sprays, *etc..* These containers should be sterile.

Where a composition/component is located in a vial, the vial is preferably made of a glass or plastic material. The vial is preferably sterilized before the composition is added to it. To avoid problems with latex-sensitive patients, vials are preferably sealed with a latex-free stopper, and the absence of latex in all packaging material is preferred. The vial may include a single dose of vaccine, or it may include more than one dose (a 'multidose' vial) *e.g.* 10 doses. Preferred vials are made of colorless glass.

A vial can have a cap (*e.g.* a Luer lock) adapted such that a pre-filled syringe can be inserted into the cap, the contents of the syringe can be expelled into the vial (*e.g.* to reconstitute lyophilised material therein), and the contents of the vial can be removed back into the syringe. After removal of the syringe from the vial, a needle can then be attached and the composition can be administered to a patient. The cap is preferably located inside a seal or cover, such that the seal or cover has to be removed before the cap can be accessed. A vial may have a cap that permits aseptic removal of its contents, particularly for multidose vials.

Where a component is packaged into a syringe, the syringe may have a needle attached to it. If a needle is not attached, a separate needle may be supplied with the syringe for assembly and use. Such a needle may be sheathed. Safety needles are preferred. 1-inch 23-gauge, 1-inch 25-gauge and 5/8-inch 25-gauge needles are typical. Syringes may be provided with peel-off labels on which the lot number, influenza season and expiration date of the contents may be printed, to facilitate record keeping. The plunger in the syringe preferably has a stopper to prevent the plunger from being accidentally removed during aspiration. The syringes may have a latex rubber cap and/or plunger. Disposable syringes contain a single dose of vaccine. The syringe will generally have a tip cap to seal the tip prior to attachment of a needle, and the tip cap is preferably made of a butyl rubber. If the syringe and needle are packaged separately then the needle is preferably fitted with a butyl rubber shield. Useful syringes are those marketed under the trade name "Tip-Lok"™.

Containers may be marked to show a half-dose volume *e.g.* to facilitate delivery to children. For instance, a syringe containing a 0.5ml dose may have a mark showing a 0.25ml volume.

Where a glass container (*e.g.* a syringe or a vial) is used, then it is preferred to use a container made from a borosilicate glass rather than from a soda lime glass.

A kit or composition may be packaged (*e.g.* in the same box) with a leaflet including details of the vaccine *e.g.* instructions for administration, details of the antigens within the vaccine, *etc.* The instructions may also contain warnings *e.g.* to keep a solution of adrenaline readily available in case of anaphylactic reaction following vaccination, *etc.*

### Methods of treatment, and Administration of the vaccine

Compositions of the invention are suitable for administration to human patients, and the invention provides a method of raising an immune response in a patient, comprising the step of administering a composition of the invention to the patient.

The invention also provides a kit or composition of the invention for use as a medicament.

The invention also provides the use of antigens from four different strains of influenza virus, in the manufacture of a medicament for raising an immune response in a patient. In some embodiments, antigens are prepared from viruses grown in cell culture. In some embodiments, an adjuvant is also used in the manufacture. In some embodiments, the antigens are not split or whole virion antigens, but are live viruses or purified surface glycoproteins. In some embodiments, the manufacture provides a vaccine containing substantially the same mass of hemagglutinin for each of the influenza virus strains.

These methods and uses will generally be used to generate an antibody response, preferably a protective antibody response. Methods for assessing antibody responses, neutralising capability and protection after influenza virus vaccination are well known in the art. Human studies have shown that antibody titers against hemagglutinin of human influenza virus are correlated with protection (a serum sample hemagglutination-inhibition titer of about 30-40 gives around 50% protection from infection by a homologous virus) [190]. Antibody responses are typically measured by hemagglutination inhibition, by microneutralisation, by single radial immunodiffusion (SRID), and/or by single radial hemolysis (SRH). These assay techniques are well known in the art.

Compositions of the invention can be administered in various ways. The most preferred immunisation route is by intramuscular injection (*e.g.* into the arm or leg), but other available routes include subcutaneous injection, intranasal [191-193], oral [194], intradermal [195,196], transcutaneous, transdermal [197], *etc.*

Vaccines prepared according to the invention may be used to treat both children and adults. Influenza vaccines are currently recommended for use in pediatric and adult immunisation, from the age of 6 months. Thus the patient may be less than 1 year old, 1-5 years old, 5-15 years old, 15-55 years old, or at least 55 years old. Preferred patients for receiving the vaccines are the elderly (*e.g.* ≥50 years old, ≥60 years old, and preferably ≥65 years), the young (*e.g.* ≥5 years old), hospitalised patients, healthcare workers, armed service and military personnel, pregnant women, the chronically ill, immunodeficient patients, patients who have taken an antiviral compound (*e.g.* an oseltamivir or zanamivir compound; see below) in the 7 days prior to receiving the vaccine, people with egg allergies and people travelling abroad. The vaccines are not suitable solely for these groups, however, and may be used more generally in a population.

Vaccines containing antigen from more than one influenza B virus strain are particularly useful for treating patients in the 0-15 years old group.

Preferred compositions of the invention satisfy 1, 2 or 3 of the CPMP criteria for efficacy. In adults (18-60 years), these criteria are: (1) ≥70% seroprotection; (2) ≥40% seroconversion; and/or (3) a GMT increase of ≥2.5-fold. In elderly (>60 years), these criteria are: (1) ≥60% seroprotection; (2) ≥30% seroconversion; and/or (3) a GMT increase of ≥2-fold. These criteria are based on open label studies with at least 50 patients.

Treatment can be by a single dose schedule or a multiple dose schedule. Multiple doses may be used in a primary immunisation schedule and/or in a booster immunisation schedule. In a multiple dose schedule the various doses may be given by the same or different routes *e.g.* a parenteral prime and mucosal boost, a mucosal prime and parenteral boost, *etc.* Administration of more than one dose (typically two doses) is particularly useful in immunologically naïve patients *e.g.* for people who have never received an influenza vaccine before, or for vaccines including a new HA subtype. Multiple doses will typically be administered at least 1 week apart (*e.g.* about 2 weeks, about 3 weeks, about 4 weeks, about 6 weeks, about 8 weeks, about 12 weeks, about 16 weeks, *etc*.).

Vaccines produced by the invention may be administered to patients at substantially the same time as (*e.g.* during the same medical consultation or visit to a healthcare professional or vaccination centre)

other vaccines *e.g.* at substantially the same time as a measles vaccine, a mumps vaccine, a rubella vaccine, a MMR vaccine, a varicella vaccine, a MMRV vaccine, a diphtheria vaccine, a tetanus vaccine, a pertussis vaccine, a DTP vaccine, a conjugated *H.influenzae* type b vaccine, an inactivated poliovirus vaccine, a hepatitis B virus vaccine, a meningococcal conjugate vaccine (such as a tetravalent A-C-W135-Y vaccine), a respiratory syncytial virus vaccine, a pneumococcal conjugate vaccine, *etc.* Administration at substantially the same time as a pneumococcal vaccine and/or a meningococcal vaccine is particularly useful in elderly patients.

Similarly, vaccines of the invention may be administered to patients at substantially the same time as (*e.g.* during the same medical consultation or visit to a healthcare professional) an antiviral compound, and in particular an antiviral compound active against influenza virus (*e.g.* oseltamivir and/or zanamivir). These antivirals include neuraminidase inhibitors, such as a (3R,4R,5S)-4-acetylamino-5-amino-3(1-ethylpropoxy)-1-cyclohexene-1-carboxylic acid or 5-(acetylamino)-4-[(aminoiminomethyl)-amino]-2,6-anhydro-3,4,5-trideoxy-D-glycero-D-galactonon-2-enonic acid, including esters thereof (*e.g.* the ethyl esters) and salts thereof (*e.g.* the phosphate salts). A preferred antiviral is (3R,4R,5S)-4-acetylamino-5-amino-3(1-ethylpropoxy)-1-cyclohexene-1-carboxylic acid, ethyl ester, phosphate (1:1), also known as oseltamivir phosphate (TAMIFLU™).

### Seasonal alternation of influenza B virus strains

One aspect of the invention provides a way of selecting the influenza B virus strain for each consecutive influenza virus season (northern of southern hemisphere) in which the strain alternates between a B/Victoria/2/87-like strain in one season and a B/Yamagata/16/88-like strain in the next season, regardless of any epidemiological analysis. These vaccines may be 3-valent or >3-valent, but they include only B/Victoria/2/87-like antigen for influenza B virus or B/Yamagata/16/88-like antigen, not both.

Thus the invention provides a method for preparing influenza vaccine in three consecutive seasons (either northern hemisphere seasons or southern hemisphere seasons), wherein the vaccine for each season contains antigen from a single influenza B virus strain, and wherein the vaccine for the first season contains antigen from a B/Victoria/2/87-like strain, the vaccine for the second season contains antigen from a B/Yamagata/16/88-like strain, and the vaccine for the third season contains antigen from a B/Victoria/2/87-like strain.

Similarly, the invention provides a method for preparing influenza vaccine in three consecutive seasons (either northern hemisphere seasons or southern hemisphere seasons), wherein the vaccine for each season contains antigen from a single influenza B virus strain, and wherein the vaccine for the first season contains antigen from a B/Yamagata/16/88-like strain, the vaccine for the second season contains antigen from a B/Victoria/2/87-like strain, and the vaccine for the third season contains antigen from a B/Yamagata/16/88-like strain.

More generally, the invention provides a method for preparing influenza vaccine for at least three consecutive seasons (either northern hemisphere seasons or southern hemisphere seasons), wherein (a) the vaccine for each season contains antigen from a single influenza B virus strain, (b) the vaccine for the first season contains antigen either from a B/Yamagata/16/88-like strain or from a B/Victoria/2/87-like strain, and (b) the vaccine for each consecutive season thereafter contains antigen from a B/Yamagata/16/88-like strain if the preceding season's vaccine included antigen from a B/Victoria/2/87-like strain, or contains antigen from a B/Victoria/2/87-like strain if the preceding season's vaccine included antigen from a B/Yamagata/16/88-like strain. Thus the antigen is selected in the first season either from a B/Yamagata/16/88-like strain or from a B/Victoria/2/87-like strain and the antigen for each season thereafter alternates between the two. This alternating cycle can be implemented for 3, 4, 5, 6, 7, 8, 9, 10, 11, 12, 13, 14, 15, 16, 17, 18, 19, 20, *etc*. consecutive seasons. Preferably the first season is 2007/08 or later.

### Multivalent vaccines including H5 strains

The invention provides 3-valent or >3-valent influenza vaccines that include antigens from at least one pandemic influenza A virus strain, at least one non-pandemic influenza A virus strain (*e.g.* from H1 and/or H3), and at least one influenza B virus strain. The pandemic strain will, based on current expectations, be a H5 strain, and so the vaccine will comprise a H5 antigen, a non-H5 influenza A antigen, and an influenza B antigen. Other features of the vaccines are as described elsewhere herein.

Thus the invention provides a 3-valent influenza virus vaccine containing antigen from one H5 influenza A virus strain, one H3N2 influenza A virus strain, and one influenza B virus strain.

The invention also provides a 3-valent influenza virus vaccine containing antigen from one H1N1 influenza A virus strain, one H5 influenza A virus strain, and one influenza B virus strain.

Current seasonal vaccines may advantageously be adapted to include antigen from a pandemic or potential-pandemic influenza virus strain. From a public heath perspective the logistics of vaccinating against the pandemic strain will be much easier than distributing separate seasonal and pandemic vaccines. Moreover, patients will receive a single vaccine to cover both the seasonal and pandemic strains, giving a better safety profile and improving patient compliance. Such vaccines can be made by manufacturing a normal trivalent seasonal vaccine and a separate monovalent pandemic vaccine, and then mixing these at the time of use, prior to administration as a single composition. As an alternative, four antigen bulks can be prepared and mixed prior to packaging and distribution.

Thus the invention also provides a 4-valent influenza virus vaccine comprising antigen from a H1N1 influenza A virus strain, a H3N2 influenza A virus strain, a pandemic influenza A virus strain (*e.g.* a H5 strain, such as H5N1), and an influenza B virus strain.

These vaccines advantageously include an adjuvant, such as an oil-in-water emulsion (as described elsewhere herein). Adjuvants are useful for improving the immune response elicited by a pandemic strain, against which patients are immunologically naïve.

In these 4-valent vaccines the antigen doses for A-H1N1, A-H3N2 and B may be substantially the same as each other *e.g.* 15µg/dose. The antigen dose for the pandemic strain may be less than that dose *e.g.* less than 10µg/dose. If the mean HA dose for A-H1N1, A-H3N2 and B is *d* µg/dose then the HA dose for the pandemic strain will be less than 0.75x *d* µg/dose *e.g.* 0.7x, 0.6x, 0.5x, 0.4x, 0.3x, 0.25x, 0.2x, or 0.1x. The individual HA doses for each of the A-H1N1, A-H3N2 and B strains is preferably within ±10% of d. In one embodiment the amount of HA per dose is in the range of 15-20µg for the three seasonal strains and about 7.5-10µg for the pandemic strain *e.g.* about 15µg/strain but about 7.5µg for the pandemic strain.

The invention also provides kits for preparing such vaccines. Thus the invention provides a kit comprising: (i) a first container containing an influenza vaccine comprising antigen from a H1N1 influenza A virus strain, a H3N2 influenza A virus strain, and an influenza B virus strain; and (ii) a second container containing an influenza vaccine comprising antigen from a pandemic strain *e.g.* a H5 strain, such as H5N1. The contents of the first and second containers can be mixed at the time of use, prior to administration to a patient as a combined vaccine. Similar kits can be used for co-administration of an influenza B virus vaccine with a normal seasonal vaccine.

In some embodiments of such kits, the first container does not include a vaccine adjuvant, but the second container does include a vaccine adjuvant e.g. an oil-in-water emulsion. After combination then the final vaccine includes the adjuvant.

The invention also provides a process for preparing a combined influenza vaccine, comprising steps of: (i) preparing bulk antigen from a H1N1 influenza A virus; (ii) preparing bulk antigen from a H3N2 influenza A virus; (iii) preparing bulk antigen from a pandemic influenza A virus, such as a H5N1 influenza A virus; (iv) preparing bulk antigen from an influenza B virus; and (v) combining and diluting the bulk antigens to give a combined vaccine having a desired concentration of each antigen. This antigen can be filled into containers and distributed for administration to patients, as described elsewhere herein.

### General

The term "comprising" encompasses "including" as well as "consisting" *e.g.* a composition "comprising" X may consist exclusively of X or may include something additional *e.g.* X + Y.

The word "substantially" does not exclude "completely" *e.g.* a composition which is "substantially free" from Y may be completely free from Y. Where necessary, the word "substantially" may be omitted from the definition of the invention.

The term "about" in relation to a numerical value x means, for example, *x*±10%.

Unless specifically stated, a process comprising a step of mixing two or more components does not require any specific order of mixing. Thus components can be mixed in any order. Where there are three components then two components can be combined with each other, and then the combination may be combined with the third component, *etc.*

Where animal (and particularly bovine) materials are used in the culture of cells, they should be obtained from sources that are free from transmissible spongiform encaphalopathies (TSEs), and in particular free from bovine spongiform encephalopathy (BSE). Overall, it is preferred to culture cells in the total absence of animal-derived materials.

Where a compound is administered to the body as part of a composition then that compound may alternatively be replaced by a suitable prodrug.

Where a cell substrate is used for reassortment or reverse genetics procedures, or for viral growth, it is preferably one that has been approved for use in human vaccine production *e.g.* as in Ph Eur general chapter 5.2.3.

### BRIEF DESCRIPTION OF DRAWINGS

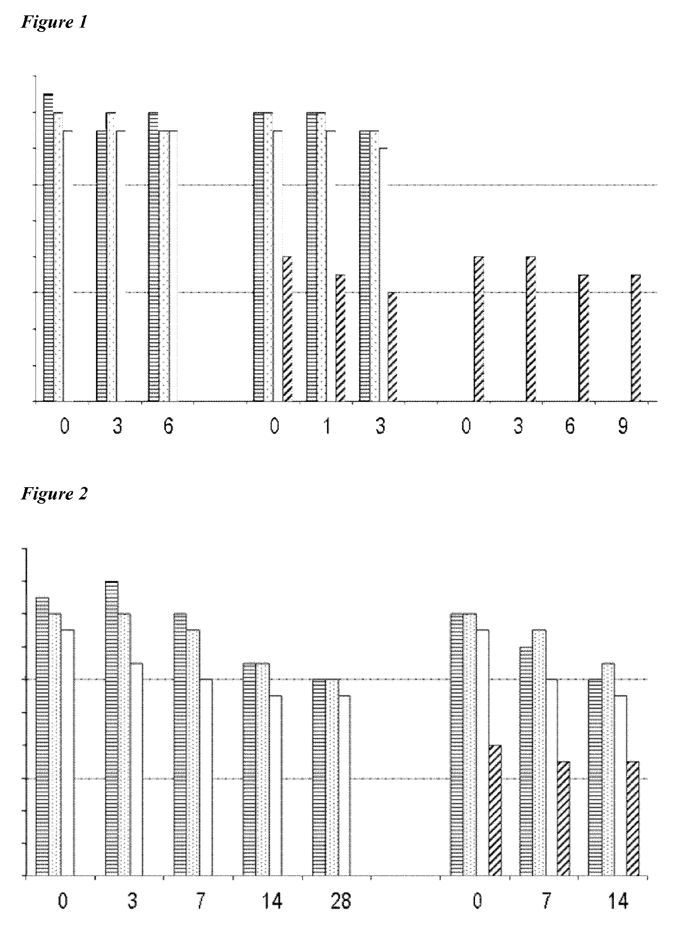

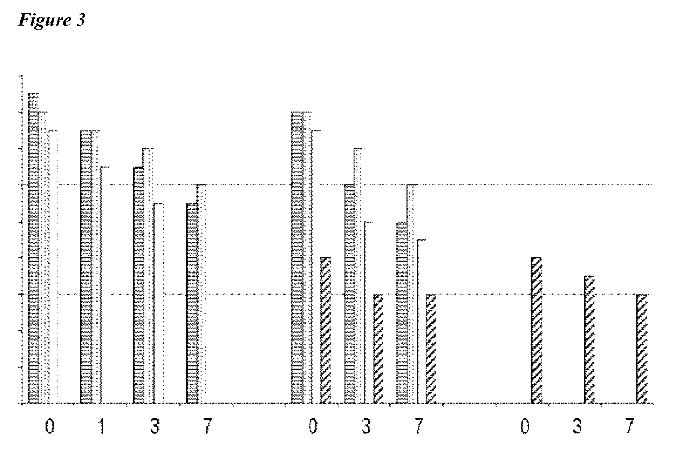

Figures 1 to 3 show vaccine stability during storage at (1) 2-8°C, (2) 23-27°C, or (3) 35-39°C. The figures on the X-axes show months in Figure 1 or days in Figures 2 & 3. The Y-axis shows HA levels, with the horizontal lines showing the stability thresholds for the seasonal antigens (upper) and the pandemic antigens (lower). In Figures 1 and 3 the three groups of bars are, from left to right: FLUAD™; 4-valent; and AFLUNOV™. In Figure 2 the two groups are FLUAD™ and 4-valent. At each timepoint data are shown, as relevant, for H1N1 (horizontal stripes), H3N2 (light shading), B (white) or H5N1 (cross-shading).

### MODES FOR CARRYING OUT THE INVENTION

### A-A-B-B vaccines

In recent years, Victoria/2/87-like and Yamagata/16/88-like lineages (`V' and 'Y' lineages) of influenza B virus have co-circulated, and their respective contributions to the overall seasonal epidemic illness burden has alternated from year to year. Vaccines in use over recent years have included either a V lineage or a Y lineage. From the 2001/02 season, the percentage of all US influenza cases that could potentially have been averted if the vaccine had included the other lineage has ranged from 0 to 29.9%, and in four of the five years starting with 2001/02 the proportion of influenza cases due to the mismatched B virus was actually greater than the proportion attributable to A/H1N1 virus:

| **Year** | **N** | **H3N2** | **H1N1** | **B all** | **B Y lineage** | **B V lineage** | **Missing coverage** |
|---|---|---|---|---|---|---|---|
| 2005 | 1019 | 55% | 13.2% | 31.5% | 5.8% | 25.6% | 25.6% |
| 2006 | | | | | (18.7%) | (81.3%) | |
| 2004 | 1075 | 65.9% | 1% | 33% | 24.5% | 8.5% | 8.5% |
| 2005 | | | | | (74.4%) | (25.6%) | |
| 2003 | 1024 | 92.6% | 0.3% | 6.9% | | 6.4% 0.4% | 6.4% |
| 2004 | | | | | (92.9%) | 7% | |
| 2002 | 699 | 20.5% | 41.1% | 38.5% | | 38.5% | |
| 2003 | | | | | 0.01%) (0.1%) | (99.9%) | - |
| 2001 | 690 | 54.0% | 4.3% | 38.7% | 8.8% | 29.9% | 29.9% |
| 2002 | | | | | (22.8%) | (77.1%) | |

| | | | | | | | |
|---|---|---|---|---|---|---|---|
| N is the number of influenza cases; percentages show the virus types responsible for those cases; the underlined figures for influenza B show the lineage that was in that season's vaccine; "Missing coverage" is the proportion of influenza cases that were caused by the influenza B virus lineage that was not in that season's vaccine. | | | | | | | |

Thus addition of the "missing" B virus lineage to form a 4-valent vaccine could have prevented up to 30% of influenza cases per season for the last 5 years, but egg-based manufacture techniques have prevented such vaccines from being produced. Cell culture techniques can overcome this problem.

### A-A-A-B vaccines

Analysis of genomes of H3N2 influenza A virus strains sampled during 1999-2004 showed that, although the majority of viruses isolated after 2002 fell into a single phylogenetic group (clade A), multiple co-circulating viral lineages were present at particular time points [1]. In the 2003-04 influenza season a major drift variant emerged in both the northern and southern hemispheres, namely the A/Fujian/411/2002-like variant. Influenza in the USA in 2001/02 was predominantly H3N2-caused, and all antigenically characterized isolates matched the A/Moscow/10/1999 vaccine strain. In the next season, when disease was dominated by H1 and influenza B, a minority of antigenically-characterized H3N2 isolates were different from the Moscow/10/1999-like vaccine strain, probably coinciding with the emergence of the Fujian strain. The 2003/04 influenza season in the northern hemisphere was also dominated by the Fujian strain, but the vaccine strain being used contained the H3N2 from the previous year (A/Panama/2007/1999). This strain was a poor antigenic match to the Fujian strain, which led to reduced vaccine effectiveness. The Fujian strain had been rejected by the FDA because it had not originally been isolated in eggs [198,199] and no antigenically-similar egg-isolated strains were available. Eliminating the need for egg isolation and/or passaging would have prevented this situation, and cell culture techniques overcome this problem. Moreover, inclusion of two H3N2 strains in an influenza vaccine would avoid any problems caused by lack of cross-reactivity between A/Moscow/10/99-like and A/Fujian/411/2002-like strains.

### Vaccines including seasonal and pandemic strains

Current seasonal influenza vaccines include hemagglutinin antigen from one H1N1 strain of influenza A virus, one H3N2 strain of influenza A virus, and one strain of influenza B virus. This seasonal combination has been supplemented by an antigen from a H5N1 strain of influenza virus i.e. a strain with the potential to cause an influenza pandemic.

A seasonal vaccine was prepared including purified surface glycoproteins from each of a A/New Caledonia H1N1 strain, a A/Wisconsin H3N2 strain and a B/Malaysia strain. The antigen dose was 30µg/ml, giving 15µg/strain/dose, but with a 15% overage. The bulk antigens were mixed at 2x strength and then diluted at a 1:1 volume ratio with MF59 oil-in-water emulsion adjuvant to give the final trivalent vaccine, which is sold under trade name FLUAD™.

A pre-pandemic vaccine was prepared based on purified surface glycoproteins from a H5N1 strain of influenza A virus. The antigen dose was 15µg/ml, giving 7.5µg/dose, but with a 15% overage. The bulk antigen at 2x strength was mixed at a 1:1 volume ratio with MF59 oil-in-water emulsion adjuvant to give the final vaccine (AFLUNOV™).

A 4-valent vaccine was prepared including the same four viral antigens. The seasonal antigens were included at the same HA concentrations as in FLUAD™ but the H5N1 antigen was included at 7.5µg/dose with no overage. The antigens were mixed at 2x strength and then diluted at a 1:1 ratio with MF59 oil-in-water emulsion adjuvant to give the final 4-valent vaccine. This vaccine is different from a simple mixture of FLUAD™ and AFLUNOV™ because the antigens are at their full dose in a 0.5ml volume, whereas a simple mixture would provide the full dose in a 1ml volume.

For stability studies vaccines were all stored at: (1) refrigerated, 2-8°C, for at least 3 months; (2) at room temperature, 23-27°C, for at least 14 days; (3) at elevated temperature, 35-39°C, at least 7 days. Antigen levels were assayed at various timepoints during storage to check for stability. The composition is deemed unstable if antigen levels drop by more than 20% below the target level during the period of the study. Figures 1 to 3 show the results.

Figure 1 shows that the antigens in the FLUAD™ formulation were stable for up to 6 months when refrigerated, and that the addition of the H5N1 strain did not cause any of the seasonal antigens to drop below the stability threshold for the 3 months of the study. Similarly, the AFLUNOV™ formulation was stable for up to 9 months, and the addition of the seasonal antigens did not cause the H5N1 antigens to drop below the stability threshold for the 3 months of the study.

Figure 2 shows that the antigens in the FLUAD™ formulation were stable for 7 days at room temperature, but that the influenza B antigen dropped below the stability threshold at 14 days. The antigens were also stable for 7 days in the presence of the H5N1 strain. The H5N1 antigen remained stable in the 4-valent composition for at least 14 days.

Figure 3 shows that the antigens in the FLUAD™ formulation were stable for 1 day at elevated temperature, but that the influenza B antigen dropped below the stability threshold at 3 days. The AFLUNOV™ formulation was stable for 7 days. The H5N1 antigen remained stable in the 4-valent composition for 7 days and it did not cause the seasonal antigens to drop below the threshold any more quickly than in the FLUAD™ formulation.

Thus a H5N1 antigen can be combined with seasonal antigens, in the presence of an adjuvant, without reducing stability of any of the 4 antigens.

In separate efficacy studies the AGRIPPAL™ seasonal vaccine and the adjuvanted AFLUNOV™ H5N1 vaccine are administered to human patients individually, concomitantly, or are mixed and then administered as a combination vaccine. Patients are split into 8 groups of 50 patients each. Groups 1 to 3 receive concomitant vaccines at time zero. Groups 4 to 6 receive the combination vaccine at time zero. Group 7 receives AFLUNOV™ at time zero. Group 8 receives AGRIPPAL™ at time zero.

At day 21, groups 1 and 4 receive no extra vaccine, but all other groups receive a second vaccine. Groups 2 and 5 receive the combination vaccine. Groups 3, 6 and 8 receive AFLUNOV™. Group 7 receives AGRIPPAL™.

At day 365 all groups receive AFLUNOV™ and immune responses are assessed.

It will be understood that the invention has been described by way of example only and modifications may be made whilst remaining within the scope and spirit of the invention.

### REFERENCES (the contents of which are hereby incorporated by reference)

[1] Holmes et al. (2005) PLoS Biol. 3(9):e300.
[2] World Health Organisation (2005) Emerging Infectious Diseases 11(10):1515-21.
[3] Rota et al. (1992) J Gen Virol 73:2737-42.
[4] GenBank sequence GI:325176.
[5] McCullers et al. (1999) J Virol 73:7343-8.
[6] GenBank sequence GI:325237.
[7] Hoffmann et al. (2002) Vaccine 20:3165-3170.
[8] Subbarao et al. (2003) Virology 305:192-200.
[9] Liu et al. (2003) Virology 314:580-590.
[10] Ozaki et al. (2004) J. Virol. 78:1851-1857.
[11] Webby et al. (2004) Lancet 363:1099-1103.
[12] WO00/60050.
[13] WO01/04333.
[14] US patent 6649372.
[15] Neumann et al. (2005) Proc Natl Acad Sci USA 102:16825-9.
[16] W02006/067211.
[17] WO01/83794.
[18] Hoffmann et al. (2000) Virology 267(2):310-7.
[19] Herlocher et al. (2004) J Infect Dis 190(9):1627-30.
[20] Le et al. (2005) Nature 437(7062):1108.
[21] Gambaryan & Matrosovich (1992) J Virol Methods 39(1-2):111-23.
[22] Mastrosovich et al. (1999) J Virol 73: 1146-55.
[23] Stevens et al. (2006) J Mol Biol 355:1143-55.
[24] Couceiro & Baum (1994) Mem Inst Oswaldo Cruz 89(4):587-91.
[25] WO02/28422.
[26] WO02/067983.
[27] WO02/074336.
[28] WO01/21151.
[29] WO02/097072.
[30] W02005/113756.
[31] Huckriede et al. (2003) Methods Enzymol 373:74-91.
[32] Treanor et al. (1996) J Infect Dis 173:1467-70.
[33] Keitel et al. (1996) Clin Diagn Lab Immunol 3:507-10.
[34] Saito et al. (2004) J Med Virol 74(2):336-43.
[35] Kistner et al. (1998) Vaccine 16:960-8.
[36] Kistner et al. (1999) Dev Biol Stand 98:101-110.
[37] Bruhl et al. (2000) Vaccine 19:1149-58.
[38] Pau et al. (2001) Vaccine 19:2716-21.
[39] *http:llwww.atcc.org*/
[40] *http:*//*locus.umdnj.edu*/
[41] WO03/076601.
[42] W02005/042728.
[43] WO03/043415.
[44] WO01/85938.
[45] WO2006/108846.
[46] WO97/37000.
[47] Brands et al. (1999) Dev Biol Stand 98:93-100.
[48] Halperin et al. (2002) Vaccine 20:1240-7.
[49] Tree et al. (2001) Vaccine 19:3444-50.
[50] EP-A-1260581 (WO01/64846).
[51] W02006/071563.
[52] W02005/113758.
[53] WO97/37001.
[54] W02006/027698.
[55] EP-B-0870508.
[56] US 5948410.
[57] Lundblad (2001) Biotechnology and Applied Biochemistry 34:195-197.
[58] Guidance for Industry: Bioanalytical Method Validation. U.S. Department of Health and Human Services Food and Drug Administration Center for Drug Evaluation and Research (CDER) Center for Veterinary Medicine (CVM). May 2001.
[59] Ji et al. (2002) Biotechniques. 32:1162-7.
[60] Briggs (1991) J Parenter Sci Technol. 45:7-12.
[61] Lahijani et al. (1998) Hum Gene Ther. 9:1173-80.
[62] Lokteff et al. (2001) Biologicals. 29:123-32.
[63] Gennaro (2000) Remington: The Science and Practice of Pharmacy. 20th edition, ISBN: 0683306472.
[64] Banzhoff (2000) Immunology Letters 71:91-96.
[65] Nony et al. (2001) Vaccine 27:3645-51.
[66] US patent 6,372,223.
[67] WO00/15251.
[68] WO01/22992.
[69] Hehme et al. (2004) Virus Res. 103(1-2):163-71.
[70] US patent 6355271.
[71] WO00/23105.
[72] US 5,057,540.
[73] WO96/33739.
[74] EP-A-0109942.
[75] WO96/11711.
[76] WO00/07621.
[77] Barr et al. (1998) Advanced Drug Delivery Reviews 32:247-271.
[78] Sjolanderet et al. (1998) Advanced Drug Delivery Reviews 32:321-338.
[79] Pizza et al. (2000) Int J Med Microbiol 290:455-461*.*
[80] WO95/17211.
[81] WO98/42375.
[82] Singh et al] (2001) J Cont Release 70:267-276.
[83] WO99/27960.
[84] US 6,090,406
[85] US 5,916,588
[86] EP-A-0626169.
[87] WO99/52549.
[88] WO01/21207.
[89] WO01/21152.
[90] WO02/072012.
[91] Signorelli & Hadden (2003) Int Immunopharmacol 3(8):1177-86.
[92] W02004/064715.
[93] Cooper (1995) Pharm Biotechnol 6:559-80.
[94] WO90/14837.
[95] Podda & Del Giudice (2003) Expert Rev Vaccines 2:197-203.
[96] Podda (2001) Vaccine 19: 2673-2680.
*[97]* Vaccine Design: The Subunit and Adjuvant Approach (eds. Powell & Newman) Plenum Press 1995 (ISBN 0-306-44867-X).
[98] Vaccine Adjuvants: Preparation Methods and Research Protocols (Volume 42 of Methods in Molecular Medicine series). ISBN: 1-59259-083-7. Ed. O'Hagan.
[99] Allison & Byars (1992) Res Immunol 143:519-25.
[100] Hariharan et al. (1995) Cancer Res 55:3486-9.
[101] US-2007/014805.
[102] Suli et al. (2004) Vaccine 22(25-26):3464-9.
[103] WO95/11700.
[104] US patent 6,080,725.
[105] W02005/097181.
[106] W02006/113373.
[107] Han et al. (2005) Impact of Vitamin E on Immune Function and Infectious Diseases in the Aged at Nutrition, Immune functions and Health EuroConference, Paris, 9-10 June 2005.
[108] US- 6630161.
[109] Hayden et al. (1998) J Clin Invest 101(3):643-9.
[110] Tassignon et al. (2005) J Immunol Meth 305:188-98.
[111] Myers et al. (1990) pages 145-156 of Cellular and molecular aspects of endotoxin reactions*.*
[112] Ulrich (2000) Chapter 16 (pages 273-282) of reference 98.
[113] Johnson et al. (1999) J Med Chem 42:4640-9.
[114] Baldrick et al. (2002) Regulatory Toxicol Pharmacol 35:398-413.
[115] US 4,680,338.
[116] US 4,988,815.
[117] WO92/15582.
[118] Stanley (2002) Clin Exp Dermatol 27:571-577.
[119] Wu et al. (2004) Antiviral Res. 64(2):79-83.
[120] Vasilakos et al. (2000) Cell Immunol. 204(1):64-74.
[121] US patents 4689338, 4929624, 5238944, 5266575, 5268376, 5346905, 5352784, 5389640, 5395937, 5482936, 5494916, 5525612, 6083505, 6440992, 6627640, 6656938, 6660735, 6660747, 6664260, 6664264, 6664265, 6667312, 6670372, 6677347, 6677348, 6677349, 6683088, 6703402, 6743920, 6800624, 6809203, 6888000 and 6924293.
[122] Jones (2003) Curr Opin Investig Drugs 4:214-218.
[123] W02004/060308.
[124] W02004/064759.
[125] US 6,924,271.
[126] US2005/0070556.
[127] US 5,658,731.
[128] US patent 5,011,828.
[129] W02004/87153.
[130] US 6,605,617.
[131] WO02/18383.
[132] W02004/018455.
[133] WO03/082272.
[134] Dyakonova et al. (2004) Int Immunopharmacol 4(13):1615-23.
[135] FR-2859633.
[136] W02006/002422.
[137] Johnson et al. (1999) Bioorg Med Chem Lett 9:2273-2278.
[138] Evans et al. (2003) Expert Rev Vaccines 2:219-229.
[139] Andrianov et al. (1998) Biomaterials 19:109-115.
[140] Payne et al. (1998) Adv Drug Delivery Review 31:185-196.
[141] De Libero et al, Nature Reviews Immunology, 2005, 5: 485-496
[142] US patent 5,936,076.
[143] Oki et al, J. Clin. Investig., 113: 1631-1640
[144] US2005/0192248
[145] Yang et al, Angew. Chem. Int. Ed., 2004, 43: 3818-3822
[146]WO2005/102049
[147] Goff et al, J. Am. Chem., Soc., 2004, 126: 13602-13603
[148] WO03/105769
[149] Thompson et al. (2003) Methods in Molecular Medicine 94:255-266.
[150] Kandimalla et al. (2003) Nucleic Acids Research 31:2393-2400.
[151] WO02/26757.
[152] WO99/62923.
[153] Krieg (2003) Nature Medicine 9:831-835.
[154] McCluskie et al. (2002) FEMS Immunology and Medical Microbiology 32:179-185.
[155] WO98/40100.
[156] US patent 6,207,646.
[157] US patent 6,239,116.
[158] US patent 6,429,199.
[159] Kandimalla et al. (2003) Biochemical Society Transactions 31 (part 3):654-658.
[160] Blackwell et al. (2003) J Immunol 170:4061-4068*.*
[161] Krieg (2002) Trends Immunol 23:64-65.
[162] WO01/95935.
[163] Kandimalla et al. (2003) BBRC 306:948-953.
[164] Bhagat et al. (2003) BBRC 300:853-861.
[165] WO03/035836.
[166] WO01/22972.
[167] Schellack et al. (2006) Vaccine 24:5461-72.
[168] Thompson et al. (2005) J Leukoc Biol 78: 'The low-toxicity versions of LPS, MPL® adjuvant and RC529, are efficient adjuvants for CD4+ T cells'.
[169] UK patent application GB-A-2220211.
[170] WO 94/21292.
[171] WO94/00153.
[172] WO95/17210.
[173] WO96/26741.
[174] WO93/19780.
[175] WO03/011223.
[176] Meraldi et al. (2003) Vaccine 21:2485-2491.
[177] Pajak et al. (2003) Vaccine 21:836-842.
[178] US-6586409.
[179] Wong et al. (2003) J Clin Pharmacol 43(7):735-42.
[180] US2005/0215517.
[181] Vaccine Design: The Subunit and Adjuvant Approach (eds. Powell & Newman) Plenum Press 1995 (ISBN 0-306-44867-X).
[182] W02005/089837.
[183] US patent 6,692,468.
[184] WO00/07647.
[185] WO99/17820.
[186] US patent 5,971,953.
[187] US patent 4,060,082.
[188] EP-A-0520618.
[189] WO98/01174.
[190] Potter & Oxford (1979) Br Med Bull 35: 69-75.
[191] Greenbaum et al. (2004) Vaccine 22:2566-77.
[192] Zurbriggen et al. (2003) Expert Rev Vaccines 2:295-304.
[193] Piascik (2003) J Am Pharm Assoc (Wash DC). 43:728-30.
[194] Mann et al. (2004) Vaccine 22:2425-9.
[195] Halperin et al. (1979) Am J Public Health 69:1247-50.
[196] Herbert et al. (1979) J Infect Dis 140:234-8.
[197] Chen et al. (2003) Vaccine 21:2830-6.
[198] Palese (2006) Emerging Infectious Diseases 12:61-65.
[199] WHO (2003) Weekly epidemiological record 78:73-80

### EMBODIMENTS OF THE INVENTION

1. A vaccine comprising antigen from at least four strains of influenza virus, wherein the vaccine is does not contain ovalbumin.
2. A vaccine comprising antigen from at least four strains of influenza virus, wherein at least one of the strains was grown in cell culture.
3. The vaccine of embodiment 1 or embodiment 2, including an adjuvant.
4. The vaccine of any preceding embodiment, wherein the antigens are neither split virions nor whole virions.
5. The vaccine of any preceding embodiment, containing substantially the same mass of hemagglutinin (HA) for each of the four influenza virus strains.
6. The vaccine of any preceding embodiment, wherein the four strains comprise two influenza A virus strains and two influenza B virus strains.
7. The vaccine of embodiment 6, comprising antigen from: (i) a H1 influenza A virus strain, such as a H1N1 strain; (ii) a H3 influenza A virus strain, such as a H3N2 strain; (iii) a B/Victoria/2/87-like influenza B virus strain; and (iv) a B/Yamagata/16/88-like influenza B virus strain.
8. The vaccine of any one of embodiments 1 to 5, wherein the four strains comprise three influenza A virus strains and one influenza B virus strain.
9. The vaccine of embodiment 8, comprising antigen from: (i) a H1 influenza A virus strain, such as a H1N1 strain; (ii) a H3 influenza A virus strain with a hemagglutinin that cross-reacts with A/Moscow/10/99; (iii) a H3 influenza A virus strain with a hemagglutinin that cross-reacts with A/Fujian/411/2002; and (iv) an influenza B virus strain from a B/Victoria/2/87-like influenza B virus strain or a B/Yamagata/16/88-like influenza B virus strain.
10. The vaccine of embodiment 8, comprising antigen from: (i) a H1 influenza A virus strain, such as a H1N1 strain; (ii) a H3 influenza A virus strain, such as a H3N2 strain; (iii) a H5 influenza A virus strain, such as a H5N1 strain; and (iv) an influenza B virus strain from a B/Victoria/2/87-like influenza B virus strain or a B/Yamagata/16/88-like influenza B virus strain.
11. The vaccine of any one of embodiments 1 to 6, comprising antigen from at least 5 influenza virus strains.
12. The vaccine of embodiment 11, comprising antigen from: (i) a H1 influenza A virus strain, such as a H1N1 strain; (ii) a H3 influenza A virus strain with a hemagglutinin that cross-reacts with A/Moscow/10/99; (iii) a H3 influenza A virus strain with a hemagglutinin that cross-reacts with A/Fujian/411/2002; (iv) an influenza B virus strain from a B/Victoria/2/87-like influenza B virus strain; and (v) a B/Yamagata/16/88-like influenza B virus strain.
13. The vaccine of embodiment 11, comprising antigen from: (i) a H1 influenza A virus strain, such as a H1N1 strain; (ii) a H3 influenza A virus strain with a hemagglutinin that cross-reacts with A/Moscow/10/99 or A/Fujian/411/2002; (iii) a H5 influenza A virus; (iv) an influenza B virus strain from a B/Victoria/2/87-like influenza B virus strain; and (v) a B/Yamagata/16/88-like influenza B virus strain.
14. The vaccine of any one of embodiments 1 to 6, comprising antigen from: (i) a H1N1 strain of influenza A virus; (ii) a A/Moscow/10/99 like H3N2 strain of influenza A virus; (iii) a A/Fujian/411/2002 like H3N2 strain of influenza A virus; (iv) a H5 influenza A virus strain, such as a H5N1 strain; (v) an influenza B virus strain from a B/Victoria/2/87-like influenza B virus strain; and (vi) a B/Yamagata/16/88-like influenza B virus strain.
15. The vaccine of any one of embodiments 1 to 5, comprising antigen from: (i) a H1N1 strain of influenza A virus; (ii) a A/Moscow/10/99 like H3N2 strain of influenza A virus; (iii) a A/Fujian/411/2002 like H3N2 strain of influenza A virus; (iv) a H5 influenza A virus strain, such as a H5N1 strain; and (v) an influenza B virus strain from a B/Victoria/2/87-like influenza B virus strain or a B/Yamagata/16/88-like influenza B virus strain.
16. The vaccine of any preceding embodiment, wherein each influenza A virus includes one or more RNA segments from a A/PR/8/34 virus.
17. The vaccine of any preceding embodiment, wherein each hemagglutinin has a binding preference for oligosaccharides with a Sia(α2,6)Gal terminal disaccharide compared to oligosaccharides with a Sia(α2,3)Gal terminal disaccharide.
18. The vaccine of any preceding embodiment, wherein each hemagglutinin has a glycoform that is not seen in chicken eggs.
19. The vaccine of any preceding embodiment, wherein the virus strains are grown in MDCK cells.
20. The vaccine of embodiment 10, wherein (i) the hemagglutinin dose for each of the H1, H3 and B strains are within 10% of the mean dose for these three strains; and (ii) the hemagglutinin dose for the H5 strain is less than 75% of the mean dose for the H1, H3 and B strains.
21. A vaccine comprising antigen from at least four strains of influenza virus, and including an adjuvant.
22. The vaccine of any one of embodiments 3 to 21, wherein the adjuvant comprises an oil-in-water emulsion comprising sub-micron droplets.
23. The vaccine of embodiment 22, wherein the oil comprises squalene.
24. The vaccine of any one of embodiments 3 to 21, wherein the adjuvant comprises a mixture of (i) an oligonucleotide including at least one CpI motif, and (ii) a polycationic oligopeptide.
25. A kit for preparing the vaccine of embodiment 22, comprising (i) a first component comprising influenza virus antigen, in a first container; and (ii) a second component comprising an oil-in-water emulsion, in a second container.
26. The vaccine of embodiment 21, wherein at least one of the strains was grown in cell culture.
27. The vaccine of embodiment 21 or embodiment 26, wherein the antigens are neither split virions nor whole virions.
28. The vaccine of embodiment 21 or embodiment 26 or embodiment 27, containing substantially the same mass of hemagglutinin (HA) for each of the four influenza virus strains.
29. A vaccine comprising antigen from at least four strains of influenza virus, wherein the antigens are neither split virions nor whole virions.
30. The vaccine of embodiment 29, where the antigens are live influenza virus or purified glycoproteins.
31. The vaccine of embodiment 29 or embodiment 30, wherein at least one of the strains was grown in cell culture.
32. The vaccine of embodiment 29 or embodiment 30 or embodiment 31, including an adjuvant.
33. The vaccine of any one of embodiments 29 to 32, containing substantially the same mass of hemagglutinin (HA) for each of the four influenza virus strains.
34. A method of raising an immune response in a patient, comprising the step of administering a vaccine of any preceding embodiment to the patient.
35. A kit comprising: (i) a first container containing an influenza vaccine comprising antigen from a H1N1 influenza A virus strain, a H3N2 influenza A virus strain, and a first influenza B virus strain; and (ii) a second container containing an influenza vaccine comprising antigen from a second influenza B virus strain, wherein one of the first and the second influenza B virus strains is a B/Victoria/2/87-like strain and the other is a B/Yamagata/16/88-like strain.
36. The use of antigens from four different strains of influenza virus, in the manufacture of a medicament for raising an immune response in a patient.
37. The use of embodiment 36, wherein the antigens are prepared from viruses grown in cell culture.
38. The use of embodiment 36 or embodiment 37, wherein the medicament is a vaccine according to any one of embodiments 1 to 33.
39. A method for immunizing a patient against influenza virus, comprising concomitantly administering to the patient (i) a first vaccine containing an influenza vaccine comprising antigen from a H1N1 influenza A virus strain, a H3N2 influenza A virus strain, and a first influenza B virus strain; and (ii) a second vaccine comprising antigen from a second influenza B virus strain, wherein one of the first and the second influenza B virus strains is a B/Victoria/2/87-like strain and the other is a B/Yamagata/16/88-like strain.

## Claims

1. A vaccine comprising antigen from at least four strains of influenza virus, wherein the four strains comprise three influenza A virus strains and one influenza B virus strain, and wherein:
a) the vaccine does not contain ovalbumin;
b) at least one of the strains was grown in cell culture;
c) the antigens are neither split virions nor whole virions; and/or
d) the vaccine includes an adjuvant.

2. The vaccine of claim 1, comprising antigen from: (i) a H1 influenza A virus strain, such as a H1N1 strain; (ii) a H3 influenza A virus strain, such as a H3N2 strain; (iii) a H5 influenza A virus strain, such as a H5N1 strain; and (iv) an influenza B virus strain from a B/Victoria/2/87-like influenza B virus strain or a B/Yamagata/16/88-like influenza B virus strain.

3. The vaccine of claim 1, comprising antigen from: (i) a H1 influenza A virus strain, such as a H1N1 strain; (ii) a H3 influenza A virus strain with a hemagglutinin that cross-reacts with A/Moscow/10/99; (iii) a H3 influenza A virus strain with a hemagglutinin that cross-reacts with A/Fujian/411/2002; and (iv) an influenza B virus strain from a B/Victoria/2/87-like influenza B virus strain or a B/Yamagata/16/88-like influenza B virus strain.

4. The vaccine of any one of claims 1-3, including an adjuvant, wherein the adjuvant comprises an oil-in-water emulsion comprising sub-micron droplets.

5. The vaccine of claim 4, wherein the oil comprises squalene.

6. The vaccine of any one of claims 1-3, including an adjuvant, wherein the adjuvant comprises a mixture of (i) an oligonucleotide including at least one CpI motif, and (ii) a polycationic oligopeptide.

7. The vaccine of any one of claims 1-6, wherein the antigens are neither split virions nor whole virions.

8. The vaccine of claim 7, wherein the antigens are live influenza virus or purified glycoproteins.

9. The vaccine of any one of claims 1-8, wherein at least one of the strains was grown in cell culture, for example, wherein the virus strains are grown in MDCK cells.

10. The vaccine of any preceding claim, containing substantially the same mass of hemagglutinin (HA) for each of the four influenza virus strains.

11. The vaccine of any one of claims 2-10, except when dependent on claim 3, wherein (i) the hemagglutinin dose for each of the H1, H3 and B strains are within 10% of the mean dose for these three strains; and (ii) the hemagglutinin dose for the H5 strain is less than 75% of the mean dose for the H1, H3 and B strains, for example, the amount of hemagglutinin per dose is about 15µg for the H1, H3 and B strains and about 7.5µg for the H5 strain.

12. A kit for preparing the vaccine of any one of claims 2-11, except when dependent on claim 3, comprising (i) a first container containing an influenza vaccine comprising antigen from a H1N1 influenza A virus strain, a H3N2 influenza A virus strain, and an influenza B virus strain; and (ii) a second container containing an influenza vaccine comprising antigen from a H5 strain, such as H5N1.

13. The use of antigens from four different strains of influenza virus in the manufacture of a 4-valent vaccine for raising an immune response in a patient, wherein the four strains comprise three influenza A virus strains and one influenza B virus strain.

14. A process for preparing the vaccine of any one of claims 2-11, except when dependent on claim 3, comprising steps of: (i) preparing bulk antigen from a H1N1 influenza A virus; (ii) preparing bulk antigen from a H3N2 influenza A virus; (iii) preparing bulk antigen from a H5N1 influenza A virus; (iv) preparing bulk antigen from an influenza B virus; and (v) combining and diluting the bulk antigens to give a combined vaccine having a desired concentration of each antigen.

15. A process for preparing a vaccine comprising the steps of: (i) growing four different strains of influenza virus, wherein the four strains comprise three influenza A virus strains and one influenza B virus strain; (ii) preparing an antigen composition from each of the viruses grown in step (i); and (iii) combining the antigen compositions with a pharmaceutical carrier, to give the vaccine.
